(19) Europäisches Patentamt
European Patent Office
Office européen des brevets

(11) EP 4 748 858 A1

(12) EUROPEAN PATENT APPLICATION
published in accordance with Art. 153(4) EPC

(43) Date of publication:
27.05.2026 Bulletin 2026/22

(51) International Patent Classification (IPC):
C07K 16/28 (2006.01)    C07K 19/00 (2006.01)
C12N 15/13 (2006.01)    C12N 15/62 (2006.01)
C12N 5/10 (2006.01)    A61K 39/00 (2006.01)
A61P 35/00 (2006.01)

(21) Application number: 24844718.7

(22) Date of filing: 19.07.2024

(86) International application number:
PCT/CN2024/106402

(87) International publication number:
WO 2025/021027 (30.01.2025 Gazette 2025/05)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 21.07.2023 CN 202310906380

(71) Applicant: Nanjing Probio Biotech Co., Ltd.
Nanjing, Jiangsu 211100 (CN)

(72) Inventors:
• LI, Yingyu
  Nanjing, Jiangsu 211100 (CN)
• XIN, Yang
  Nanjing, Jiangsu 211100 (CN)
• LI, Lianqu
  Nanjing, Jiangsu 211100 (CN)
• QI, Zeng
  Nanjing, Jiangsu 211100 (CN)
• JIA, Wenshuang
  Nanjing, Jiangsu 211100 (CN)
• ZHOU, Yi
  Nanjing, Jiangsu 211100 (CN)
• CHEN, Li
  Nanjing, Jiangsu 211100 (CN)
• LIANG, Yu
  Nanjing, Jiangsu 211100 (CN)

(74) Representative: Synergy IP Group AG
Leonhardsgraben 52
Postfach
4001 Basel (CH)

Remarks:
The complete document including Reference Table(s) and the Sequence Listing(s) can be downloaded from the EPO website

(54) **ANTIBODY TARGETING HUMAN CD7 OR ANTIGEN-BINDING FRAGMENT THEREOF AND USE THEREOF**

(57) An antibody targeting human CD7 or an antigen fragment thereof and the use thereof. The antibody and CD7-CAR based on the antibody fragment have a very strong affinity for a CD7 antigen molecule, and a blocking molecule containing the antibody can almost completely block the expression of the CD7 molecule on the cell surface without affecting the normal amplification of T cells, thereby effectively avoiding the fratricide killing of CD7-CAR-T cells. The CD7-CAR-T cells, which are constructed on the basis of a single domain antibody targeting human CD7, have a strong killing effect with regard to target cells.

EP 4 748 858 A1

**Description**

CROSS-REFERENCE TO RELATED APPLICATIONS

The present application claims priority to the Chinese Patent Application No. 202310906380.4 filed with the China National Intellectual Property Administration on July 21, 2023, and entitled "ANTIBODY TARGETING HUMAN CD7 OR ANTIGEN-BINDING FRAGMENT THEREOF AND USE THEREOF", which is incorporated herein by reference in its entirety.

TECHNICAL FIELD

The present invention belongs to the fields of tumour immunotherapy and molecular immunology, and specifically relates to a single domain antibody targeting human CD7, a CD7-CAR-T cell comprising the single domain antibody, and a preparation method therefor and use thereof.

BACKGROUND

Tumour immunotherapy has become one of the most important means of tumour treatment. The immune system can effectively attack cancer cells only after recognizing them. Carcinogenesis results from the loss of normal regulatory functions in normal somatic cells and the accumulation of genetic mutations to a certain extent. However, cancer cells can disguise themselves as normal somatic cells, cleverly evading attacks from the immune system.

Tumour-associated antigens (TAAs) refer to self-antigens expressed by tumour cells. These antigens exist partially in normal host cells and are mainly derived from gene amplification or post-translational modification, but they tend to be highly expressed or specifically expressed in tumour cells. A large number of tumour-associated antigens and tumour-specific antigens have been discovered so far, and targeted therapy targeting TAAs is an important approach in cancer treatment. Many drugs for cancer immunotherapy based on this mechanism are already available on the market and have demonstrated significant clinical efficacy.

In general, antibody-mediated TAA recognition can cleverly direct NK cells or T cells to target tumour cells with high TAA expression. Antibodies targeting TAAs (e.g., rituximab and trastuzumab) can not only directly kill tumour cells through the ADCC effect, but also serve as diagnostic markers or innovatively enhance the targeting of traditional cancer therapies.

Adoptive immunotherapy is a transformative therapeutic method in haematology, which involves genetically engineering T cells to express and synthesize chimeric antigen receptors (CARs). Chimeric antigen receptors (CARs) are engineered receptors that can redirect immune cells to target cancer cells. CAR-T cells have achieved remarkable outcomes in patients with hematologic malignancies, and the FDA has approved 6 types of CAR-T cells for the treatment of relapsed or refractory B-cell malignancies, namely: Abecma, Breyanzi, Carvykti, Kymriah, Tecartus and Yescarta. The components of these CARs (including the extracellular antigen-binding domain, hinge and transmembrane region, co-stimulatory domain and activation domain) are systematically designed to optimize cell activation and enhance cell persistence. Using different domains or making minor modifications to domain sequences can significantly alter the efficacy of CAR-T cells. Despite the success of CAR-T therapy in haematological malignancies to date, many patients have experienced CAR-T cell-related toxicities and relapsed after CAR-T cell therapy, which may be associated with the binding epitopes of CARs, suggesting the necessity to develop antibodies targeting diverse epitopes. The next-generation CAR-T cell therapy aims to reduce toxicity and prevent relapse by targeting antigens, regulating the assembly or activation of CAR components, preventing anti-CAR immunity and/or protecting cells to respond to their surrounding environment. Although CAR-T cells approved by the FDA target two B-cell antigens, CD19 and B-cell maturation antigen (BCMA), other CAR-T cells targeting B-cell malignancies, other haematological cancers, and solid tumours are rapidly emerging. Compared to B-cell malignancies, the use of first-line cancer treatments (including chemotherapy) in patients with T-cell malignancies only achieves limited clinical responses, resulting in poor prognosis for these patients. Given the clinical success of CAR-T therapy in B-cell malignancies, there is an expectation that the CAR-T therapy can also improve clinical outcomes for patients with T-cell malignancies. However, CAR-T therapy for T-cell tumours remains considerably challenging. The first challenge is the lack of T-cell tumour-specific antigens for CAR-T targeting. Secondly, fratricide killing, T-cell aplasia, and product contamination by malignant T cells during the production of autologous CAR-T cells are all among the most critical challenges in CAR-T therapy for T-cell malignancies, all of which require further in-depth research.

CD7 is a transmembrane glycoprotein belonging to the immunoglobulin superfamily and exists as a homodimer on the cell surface of most mature peripheral blood T cells, all thymocytes and natural killer (NK) cells, and is also expressed on myeloid cell lineages and T/B cells that generate immature progenitor cells. CD7 is one of the primary markers during T-cell maturation. Therefore, it is used as a diagnostic marker for detecting primary T-cell cancers. CD7 is highly expressed in T-cell acute lymphoblastic leukaemia (T-ALL) and subsets of peripheral T-cell lymphoma. In contrast, the normal expression of CD7 is mainly restricted to T cells and natural killer (NK) cells, thereby reducing the risk of off-target organ toxicity. CD7 has been used as a target for immunotoxin antibodies in some clinical studies involving patients with T-cell malignancies due to its internalization properties. Although no severe permanent adverse effects associated with CD7 antibodies are observed, the tumour response remains limited. Therefore, the efficacy of CD7-directed cytotoxicity can be enhanced by replacing monoclonal antibodies with autologous CAR-T cells, which will improve the therapeutic effect of CD7-targeted therapy in patients with T-cell malignancies. (Gomes-Silva D, Srinivasan M, Sharma S, Lee CM, Wagner DL, Davis TH, et al. CD7-edited T cells expressing a CD7-specific CAR for the therapy of T-cell malignancies. Blood. 2017;130 (3): 285-296; Sempowski GD, Lee DM, Kaufman RE, Haynes BF. Structure and function of the CD7 molecule. Crit Rev Immunol. 1999; 19 (4): 331-348; Safarzadeh Kozani P, Safarzadeh Kozani P, Rahbarizadeh F. CAR-T cell therapy in T-cell malignancies: Is success a low-hanging fruit? Stem Cell Res Ther. 2021 Oct 7;12 (1): 527).

Compared with CAR-T therapy for B-ALL, the application of CD7-CAR-T cell-targeted therapy for T-cell acute lymphoblastic leukemia and CD7-positive lymphomas still faces significant technical challenges. This is because both normal effector T cells and T-cell tumours express the CD7 antigen, which leads to the "fratricide killing" effect of CD7-CAR-T cells. With current technologies, the *in vitro* preparation of CD7-CAR-T cells often fails to succeed due to the presence of this "suicide" effect. Therefore, there is a need to develop a novel CD7 antibody or an antigen fragment thereof, which blocks CD7 expression on T cells, and when expressed on CD7-CAR-T cells, prevents CD7-CAR-T cells from undergoing suicide during the preparation, enabling their normal expansion *in vitro* while maintaining killing ability against CD7-positive target cells.

SUMMARY

To solve technical problems in the prior art, the inventor has, through in-depth research, prepared and selected a unique anti-human CD7 single domain antibody sequence. Using CDR grafting technology, the CDRs of a positive single domain antibody are grafted into a human natural germline sequence. Through combinatorial design, a DNA fragment of a humanized antibody heavy chain variable region is obtained, and serves as an antigen recognition element for the construction of CD7-CAR-T in the present invention. Meanwhile, a CD7 vector that effectively blocks the expression of a CD7 molecule on the surface of T cells is constructed, thereby eliminating the "fratricide killing" effect of CD7-CAR-T cells and facilitating the *in vitro* preparation and production of CD7-CAR-T cells. The CD7-CAR-T cell constructed with the single domain antibody targeting human CD7 provided by the present invention has a superior *in vitro* killing effect on the target cell Jurkat/Luc compared to the positive control VHH6 CAR-T cell. Specifically, the present invention includes the following contents.

In a first aspect, the present invention provides an antibody or an antigen-binding fragment thereof that specifically binds to a CD7 antigen, and comprises a heavy chain variable region, wherein the heavy chain variable region comprises complementarity determining region 1 (CDR 1), complementarity determining region 2 (CDR2), and complementarity determining region 3 (CDR3), wherein:

> the CDR1 comprises a sequence as set forth in SEQ ID NO: 18, the CDR2 comprises a sequence as set forth in SEQ ID NO: 19, and the CDR3 comprises a sequence as set forth in SEQ ID NO: 20;
> the CDR1 comprises a sequence as set forth in SEQ ID NO: 42, the CDR2 comprises a sequence as set forth in SEQ ID NO: 43, and the CDR3 comprises a sequence as set forth in SEQ ID NO: 44;
> the CDR1 comprises a sequence as set forth in SEQ ID NO: 2, the CDR2 comprises a sequence as set forth in SEQ ID NO: 3, and the CDR3 comprises a sequence as set forth in SEQ ID NO: 4;
> the CDR1 comprises a sequence as set forth in SEQ ID NO: 6, the CDR2 comprises a sequence as set forth in SEQ ID NO: 7, and the CDR3 comprises a sequence as set forth in SEQ ID NO: 8;
> the CDR1 comprises a sequence as set forth in SEQ ID NO: 10, the CDR2 comprises a sequence as set forth in SEQ ID NO: 11, and the CDR3 comprises a sequence as set forth in SEQ ID NO: 12;
> the CDR1 comprises a sequence as set forth in SEQ ID NO: 14, the CDR2 comprises a sequence as set forth in SEQ ID NO: 15, and the CDR3 comprises a sequence as set forth in SEQ ID NO: 16;
> the CDR1 comprises a sequence as set forth in SEQ ID NO: 22, the CDR2 comprises a sequence as set forth in SEQ ID NO: 23, and the CDR3 comprises a sequence as set forth in SEQ ID NO: 24;
> the CDR1 comprises a sequence as set forth in SEQ ID NO: 26, the CDR2 comprises a sequence as set forth in SEQ ID NO: 27, and the CDR3 comprises a sequence as set forth in SEQ ID NO: 28;
> the CDR1 comprises a sequence as set forth in SEQ ID NO: 30, the CDR2 comprises a sequence as set forth in SEQ ID NO: 31, and the CDR3 comprises a sequence as set forth in SEQ ID NO: 32;
> the CDR1 comprises a sequence as set forth in SEQ ID NO: 34, the CDR2 comprises a sequence as set forth in SEQ ID NO: 35, and the CDR3 comprises a sequence as set forth in SEQ ID NO: 36;
> the CDR1 comprises a sequence as set forth in SEQ ID NO: 38, the CDR2 comprises a sequence as set forth in SEQ ID NO: 39, and the CDR3 comprises a sequence as set forth in SEQ ID NO: 40; or

the CDR1 comprises a sequence as set forth in SEQ ID NO: 46, the CDR2 comprises a sequence as set forth in SEQ ID NO: 47, and the CDR3 comprises a sequence as set forth in SEQ ID NO: 48;
the CDR1, the CDR2, and the CDR3 each comprise up to 3 amino acid substitutions, insertions, or deletions.

In certain embodiments, the heavy chain variable region comprises CDR1, CDR2, and CDR3, wherein:

the CDR1 comprises a sequence as set forth in SEQ ID NO: 18, the CDR2 comprises a sequence as set forth in SEQ ID NO: 19, and the CDR3 comprises a sequence as set forth in SEQ ID NO: 20;
the CDR1 comprises a sequence as set forth in SEQ ID NO: 42, the CDR2 comprises a sequence as set forth in SEQ ID NO: 43, and the CDR3 comprises a sequence as set forth in SEQ ID NO: 44;
the CDR1 comprises a sequence as set forth in SEQ ID NO: 2, the CDR2 comprises a sequence as set forth in SEQ ID NO: 3, and the CDR3 comprises a sequence as set forth in SEQ ID NO: 4;
the CDR1 comprises a sequence as set forth in SEQ ID NO: 6, the CDR2 comprises a sequence as set forth in SEQ ID NO: 7, and the CDR3 comprises a sequence as set forth in SEQ ID NO: 8;
the CDR1 comprises a sequence as set forth in SEQ ID NO: 10, the CDR2 comprises a sequence as set forth in SEQ ID NO: 11, and the CDR3 comprises a sequence as set forth in SEQ ID NO: 12;
the CDR1 comprises a sequence as set forth in SEQ ID NO: 14, the CDR2 comprises a sequence as set forth in SEQ ID NO: 15, and the CDR3 comprises a sequence as set forth in SEQ ID NO: 16;
the CDR1 comprises a sequence as set forth in SEQ ID NO: 22, the CDR2 comprises a sequence as set forth in SEQ ID NO: 23, and the CDR3 comprises a sequence as set forth in SEQ ID NO: 24;
the CDR1 comprises a sequence as set forth in SEQ ID NO: 26, the CDR2 comprises a sequence as set forth in SEQ ID NO: 27, and the CDR3 comprises a sequence as set forth in SEQ ID NO: 28;
the CDR1 comprises a sequence as set forth in SEQ ID NO: 30, the CDR2 comprises a sequence as set forth in SEQ ID NO: 31, and the CDR3 comprises a sequence as set forth in SEQ ID NO: 32;
the CDR1 comprises a sequence as set forth in SEQ ID NO: 34, the CDR2 comprises a sequence as set forth in SEQ ID NO: 35, and the CDR3 comprises a sequence as set forth in SEQ ID NO: 36;
the CDR1 comprises a sequence as set forth in SEQ ID NO: 38, the CDR2 comprises a sequence as set forth in SEQ ID NO: 39, and the CDR3 comprises a sequence as set forth in SEQ ID NO: 40; or
the CDR1 comprises a sequence as set forth in SEQ ID NO: 46, the CDR2 comprises a sequence as set forth in SEQ ID NO: 47, and the CDR3 comprises a sequence as set forth in SEQ ID NO: 48.

In certain embodiments, the CDR region sequences are selected from any one of the following groups:

the CDR1 comprises a sequence as set forth in SEQ ID NO: 18, the CDR2 comprises a sequence as set forth in SEQ ID NO: 19, and the CDR3 comprises a sequence as set forth in SEQ ID NO: 20;
the CDR1 comprises a sequence as set forth in SEQ ID NO: 42, the CDR2 comprises a sequence as set forth in SEQ ID NO: 43, and the CDR3 comprises a sequence as set forth in SEQ ID NO: 44;
the CDR1 comprises a sequence as set forth in SEQ ID NO: 14, the CDR2 comprises a sequence as set forth in SEQ ID NO: 15, and the CDR3 comprises a sequence as set forth in SEQ ID NO: 16.

In certain embodiments, the heavy chain variable region comprises framework regions (FRs) of an alpaca antibody or a human antibody.
In certain embodiments, the antibody or the antigen-binding fragment thereof is selected from the group consisting of a whole antibody, Fab, Fab', (Fab')2, an Fv fragment, scFv, di-scFv, and a single domain antibody (sdAb). In a specific embodiment, the antibody or the antigen-binding fragment thereof is a single domain antibody.
In certain embodiments, the antibody or the antigen-binding fragment thereof is a chimeric antibody, an Fc fusion antibody, or a humanized antibody. In certain embodiments, the antibody or the antigen-binding fragment thereof is a humanized antibody. In a specific embodiment, the antibody or the antigen-binding fragment thereof is humanized sdAb.
In certain embodiments, the antibody or the antigen-binding fragment thereof comprises a mutant amino acid sequence having at least 80% identity to an amino acid sequence as set forth in any one of SEQ ID NOs: 17, 41, 1, 5, 9, 13, 21, 25, 29, 33, 37, 45, and 49-51.
In a second aspect, the present invention provides a CD7 blocking molecule, which comprises the antibody or the antigen-binding fragment thereof according to the first aspect, and an endoplasmic reticulum localization domain, wherein the blocking molecule is capable of reducing the expression of a CD7 molecule on the cell surface.
In certain embodiments, the CD7 blocking molecule comprises one or more identical single domain antibodies, which are connected via a linker. In certain embodiments, the linker sequence is the sequence as set forth in SEQ ID NO: 64.
In a specific embodiment, the CD7 blocking molecule comprises two identical single domain antibodies, which are connected via a linker.

In certain embodiments, the sequence of the endoplasmic reticulum localization domain comprises a sequence as set forth in SEQ ID NO: 63.

In certain embodiments, the CD7 blocking molecule further comprises a signal peptide sequence. In certain embodiments, the signal peptide sequence comprises a sequence as set forth in SEQ ID NO: 53.

In certain embodiments, the amino acid sequence of the CD7 blocking molecule comprises a sequence as set forth in SEQ ID NO: 52 or a mutant sequence having at least 80% identity thereto.

In a third aspect, the present invention provides a chimeric antigen receptor (CAR), which comprises:

1) an antigen-binding domain that recognizes a CD7 antigen, wherein the antigen-binding domain comprises the antibody or the antigen-binding fragment thereof according to the first aspect;
2) a transmembrane domain; and
3) an intracellular signalling domain.

In certain embodiments, the chimeric antigen receptor comprises a hinge region between the antigen-binding domain and the transmembrane domain, wherein the hinge region comprises a hinge region derived from one or more proteins selected from the group of: CD28, IgG1, IgG4, IgD, 4-1BB, CD4, CD27, CD7, CD8 alpha, PD-1, ICOS, OX40, NKG2D, NKG2C, FcεRIγ, BTLA, GITR, DAP10, CD40L, TIM1, CD226, SLAM, CD30, and LIGHT.

In certain embodiments, the hinge region comprises a hinge region derived from CD28. In a specific embodiment, the hinge region sequence comprises a sequence as set forth in SEQ ID NO: 55.

In certain embodiments, the transmembrane domain of the chimeric antigen receptor comprises a transmembrane domain derived from one or more proteins selected from the group of: CD8, CD28, 4-1BB, CD4, CD27, CD7, PD-1, TRAC, TRBC, CD3e, CD3, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, TIM1, CD226, DR3, CD45, CD80, CD86, CD9, CD16, CD22, CD33, CD37, CD64, CD134, CD137, CD154, and SLAM.

In certain embodiments, the transmembrane domain comprises a transmembrane domain derived from CD28. In certain embodiments, the transmembrane domain comprises a transmembrane domain derived from CD8. In a specific embodiment, the transmembrane region sequence comprises a sequence as set forth in SEQ ID NO: 56.

In certain embodiments, the intracellular signal transduction domain of the chimeric antigen receptor comprises an intracellular signal transduction domain derived from one or more proteins selected from the group of: CD8, CD3ζ, CD3δ, CD3γ, CD3ε, FcγRI-γ, FcγRIII-γ, FcεRIβ, FcεRIγ, DAP10, DAP12, CD32, CD79a, CD79b, CD28, CD3C, CD4, b2c, CD137 (4-1BB), ICOS, CD27, CD28δ, CD80, NKp30, and OX40.

In certain embodiments, the intracellular signal transduction domain comprises CD3ζ. In a specific embodiment, the signal transduction domain comprises a sequence as set forth in SEQ ID NO: 58.

In certain embodiments, the chimeric antigen receptor further comprises an intracellular co-stimulatory signalling domain.

The intracellular co-stimulatory signalling domain comprises an intracellular co-stimulatory signalling domain derived from one or more proteins selected from the group of: CD28, CD137, CD27, CD2, CD7, CD8, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, ligand for CD83, CD40, and MyD88.

In certain embodiments, the intracellular co-stimulatory signalling domain comprises a sequence derived from the intracellular region of CD28. In certain embodiments, the intracellular co-stimulatory signalling domain comprises a sequence derived from the intracellular region of 4-1BB. In a specific embodiment, the intracellular costimulatory signalling domain comprises a sequence as set forth in SEQ ID NO: 57.

In certain embodiments, the structure of the chimeric antigen receptor comprises multiple domains, such as: signal peptide-VHH-hinge region-transmembrane region-intracellular co-stimulatory signalling domain-intracellular signal transduction domain, wherein the domains are independently connected via a linker or a peptide bond. The VHH may be one or more VHHs connected via a linker.

In certain embodiments, the signal peptide sequence is a signal peptide of a protein selected from the group of: CD8, CD28, GM-CSF, CD4, CD137, or a combination thereof. In a specific embodiment, the signal peptide sequence comprises a sequence as set forth in SEQ ID NO: 53.

In certain embodiments, the chimeric antigen receptor comprises an amino acid sequence as set forth in any one of SEQ ID NOs: 59-61.

In a fourth aspect, the present application provides an isolated nucleic acid molecule encoding the antibody or the antigen-binding fragment thereof according to the first aspect of the present invention, or the blocking molecule according to the second aspect, or the chimeric antigen receptor according to the third aspect.

In a fifth aspect, the present application provides a vector comprising the nucleic acid molecule according to the fourth aspect.

In certain embodiments, the vector is DNA, RNA, a plasmid, a lentiviral vector, an adenoviral vector, AAV, a retroviral vector, a transposon, or a combination thereof.

In certain embodiments, the vector is a lentiviral vector.

In a sixth aspect, the present application provides a host cell comprising the vector according to the fifth aspect.

In a seventh aspect, the present application provides an engineered immune cell comprising the antibody or the antigen-binding fragment thereof according to the first aspect of the present invention, or the CD7 blocking molecule according to the second aspect, or the chimeric antigen receptor according to the third aspect.

In certain embodiments, the immune cell is selected from: at least one of a leukocyte, a monocyte, a macrophage, a dendritic cell, a mast cell, a neutrophil, a basophil, an eosinophil, an $\alpha\beta$ T cell, a $\gamma\delta$ T cell, a natural killer (NK) cell, a natural killer T (NKT) cell, a B cell, an innate lymphoid cell (ILC), a cytokine-induced killer (CIK) cell, a cytotoxic T lymphocyte (CTL), a lymphokine-activated killer (LAK) cell, a T lymphocyte, a peripheral blood mononuclear cell, and a hematopoietic stem cell.

In an eighth aspect, the present application provides a pharmaceutical composition comprising: the antibody or antigen-binding fragment thereof according to the first aspect of the present invention, or the blocking molecule according to the second aspect, or the chimeric antigen receptor according to the third aspect, or the engineered immune cell according to the seventh aspect, and/or a pharmaceutically acceptable carrier.

In a ninth aspect, the present application provides use of the antibody or the antigen-binding fragment thereof according to the first aspect, or the blocking molecule according to the second aspect, or the engineered immune cell according to the seventh aspect, or the composition according to the eighth aspect in the treatment of a tumour or cancer.

In certain embodiments, the cancer or tumour refers to a cancer or tumour related to CD7 expression.

In certain embodiments, the cancer or tumour is a haematological malignancy.

In certain embodiments, the haematological malignancy is a T cell-related tumour, wherein the T cell-related tumour includes leukaemia, lymphoma, and myeloma.

In a tenth aspect, the present application provides a method for preparing an engineered immune cell, wherein the method comprises the following steps:

(1) providing a cell to be used for expressing a chimeric antigen receptor, wherein the immune cell comprises the blocking molecule according to the second aspect; and
(2) introducing a nucleic acid molecule to the cell obtained in step (1), wherein the nucleic acid molecule expresses the chimeric antigen receptor according to the third aspect.

Beneficial Effects of Present Invention:

The antibody or the antigen-binding fragment thereof provided by the present invention has a very strong affinity for the CD7 antigen molecule. Meanwhile, the blocking molecule of the present invention can almost completely block the expression of the CD7 molecule on the cell surface after transfection, without affecting the normal expansion of T cells, thereby effectively avoiding the fratricide killing of CD7-CAR-T cells. The CD7-CAR-T cells constructed with a humanized CD7 nanobody sequence in the present invention can further reduce the immunogenicity of CD7-CAR-T cells constructed with an alpaca-derived CD7 antibody sequence *in vivo,* thereby improving the clinical therapeutic effect. In addition, the CD7-CAR-T cells constructed with the single domain antibody targeting human CD7 provided by the present invention has a strong killing effect on target cells, which is superior to that of the positive control VHH6 CAR-T cells, thereby providing a beneficial CAR-T cell for the clinical application of cell therapy.

BRIEF DESCRIPTION OF THE DRAWINGS

FIG. 1 shows the assay results of the binding of the antibodies of the present application to human CD7 protein.
FIG. 2 shows the assay results of the binding of the antibodies of the present application to monkey CD7 protein.
FIG. 3 shows the assay results of the binding of the antibodies of the present application to mouse CD7 protein.
FIG. 4 shows the FACS assay results of the binding of the antibodies of the present application to cells expressing human CD7 protein.
FIG. 5 shows the assay results of the affinity of the humanized antibodies of the present application for human CD7 protein,
in which panels A-G show the affinity assay signal graphs of three antibodies and their corresponding humanized antibodies for human CD7 protein, respectively.
FIG. 6 shows the assay results of the affinity of the humanized antibodies of the present application for monkey CD7 protein,

in which panels A-G show the affinity assay signal graphs of three humanized antibodies for monkey CD7 protein, respectively.

FIG. 7 shows a schematic diagram of the structure of CD7 blocking molecules in some examples of the present application.

FIG. 8 shows the blocking effect of the CD7 blocking molecules of the present application on CD7 expression in T cells, in which panels A-F show the assay results of CD7 expression blocking on the cell surface by three different CD7 blocking molecules under conditions of different viral titres.

FIG. 9 shows the blocking effect of the AHP19485-VHH1 molecule on CD7 expression in T cells.

FIG. 10 shows the design concept of the *in vitro* activity experiment on the CD7-CAR-T cells of the present application.

FIG. 11 shows a schematic diagram of the structure of the CARs in some examples of the present application.

FIG. 12 shows the blocking effect of the AHP19485-VHH1 molecule on CD7 expression in T cells.

FIG. 13 shows the assay results of the positive rate of CD7 expression on the CD7-CAR-T cells constructed in the present application.

FIG. 14 shows the *in vitro* target cell killing effect of the CD7-CAR-T cells constructed in the present application, in which panel A shows the killing effect of CAR-T cells with different CD7 antibody molecules on Jurkat target cells, and panel B shows the killing effect of CAR-T cells with different CD7 antibody molecules on CCRF-CEM target cells.

DETAILED DESCRIPTION

The technical solutions of the present invention will be further described in detail with reference to the following specific examples. It should be understood that the following examples are merely exemplary illustrations and explanations of the present invention and should not be construed as limiting the scope of protection of the present invention. All techniques implemented on the basis of the content described above of the present invention are encompassed within the scope of protection of the present invention.

Unless otherwise stated, the starting materials and reagents used in the following examples are all commercially available products or can be prepared by using known methods. In the following examples, experimental methods without specific conditions specified were generally performed according to conventional conditions, such as conditions described in Sambrook, et al., Molecular Cloning: A Laboratory Manual (New York: Cold Spring Harbor Laboratory Press, 1989), or conditions recommended by the manufacturer.

Unless defined otherwise, all technical and scientific terms used herein have the same meaning as commonly understood by those of ordinary skill in the art. Although only preferred methods and materials are described herein, any methods and materials similar or equivalent to those described herein can be used in the practice or testing of the present invention. All documents mentioned herein are incorporated by reference to disclose and describe the methods and/or materials related to the documents. In case of conflict with any incorporated document, the specification shall prevail.

Definitions and Description:

The term "antibody" used herein is used in its broadest sense and includes immunoglobulins or other types of molecules that comprise one or more antigen-binding domains that specifically bind to an antigen, and refers to a protein or polypeptide that exhibits binding specificity for a particular antigen. Specific examples of antibodies may include intact antibodies (e.g., classical four-chain antibody molecules), single chain antibodies, single domain antibodies, multispecific antibodies, etc. A classical antibody molecule is generally a tetramer composed of two identical heavy chains and two identical light chains, which are interconnected via disulfide bonds. Based on differences in the conservation of amino acid sequences, the heavy chains and light chains are each divided into two regions: a variable region (V) located at the amino terminus and a constant region (C) located at the carboxyl terminus. The variable region is used for recognizing and binding to antigens, whereas the constant region (e.g., the Fc fragment) is used for initiating downstream effector functions, such as antibody-dependent cell-mediated cytotoxicity (ADCC). In each of the variable regions of the heavy chains and light chains, there are three local regions with a higher degree of variation in amino acid composition and arrangement sequence, which are the key positions for the binding of antibodies to antigens, and thus are also called complementarity determining regions (CDRs). The amino acid sequences of CDRs can be easily determined using numbering schemes recognized in the art, e.g., Kabat, Chothia, IMGT, AbM, or Contact.

Based on the amino acid sequences of the constant regions of antibody heavy chains, antibodies can be classified into five major distinct types: IgA, IgD, IgE, IgG, and IgM. These antibody types may be further divided into subclasses based on differences in the size of the hinge region, the position of interchain disulfide bonds, and molecular weight, e.g., IgG1, IgG2a, IgG2b, IgG3, etc. Based on differences in the amino acid composition and arrangement of the constant regions of antibody light chains, light chains can be classified into two types: $\kappa$ and $\lambda$. The subunit structures and three-dimensional conformations of different classes of immunoglobulins are known in the art.

The variable regions of antibody heavy and light chains generally comprise 3 complementarity determining regions (CDRs) and 4 framework regions (FRs). The complementarity determining regions are connected by framework regions. During antibody recognition, the FR molecules fold to bring the CDR molecules close to each other. The complementarity determining regions are the binding sites for antibodies or antigen-binding fragments to antigens. Therefore, the sequences of complementarity determining regions determine the specificity of antibodies. As understood in the art, an antibody is a glycoprotein comprising at least two heavy (H) chains and two light (L) chains interconnected via disulfide bonds, or an antigen-binding portion thereof. The heavy chain comprises a heavy chain variable region (VH) and a heavy chain constant region (CH). The light chain comprises a light chain variable region (VL) and a light chain constant region (CL). The variable regions of the heavy and light chains comprise framework regions (FRs) and complementarity determining regions (CDRs). The four FRs are relatively conserved, whereas the CDR regions (i.e., CDR1, CDR2, and CDR3) comprise hypervariable regions.

In the present application, the term "CDR" generally refers to a complementarity determining region, which is primarily responsible for binding to antigenic epitopes. The CDRs of the heavy chain are generally referred to as CDR1, CDR2, and CDR3, which are numbered sequentially from the N-terminus. The 3 CDR fragments, together with the framework regions, constitute the heavy chain variable region, namely the VHH segment. A variety of schemes are provided for the definition of the CDR regions, e.g., the Chothia scheme, the Kabat scheme, etc. In the present application, the CDRs can be divided according to the Kabat scheme.

The term "antigen-binding fragment" herein refers to a polypeptide fragment that comprises a portion of an intact antibody, such as the antigen-binding region or variable region of an intact antibody, has the property of being able to specifically target CD7, and includes Fab, Fab', an Fv fragment, F(ab')2, scFv, di-scFv and/or sdAb, etc. Preferably, the antigen-binding fragment comprises at least one CDR from the heavy chain variable region and/or the light chain variable region of an antibody; more preferably, the antigen-binding fragment may comprise CDRs 1-3 from the heavy chain variable region and/or CDRs 1-3 from the light chain variable region. Antigen-binding fragments can be prepared by a variety of techniques, including but not limited to the hydrolytic digestion of intact antibody proteins, or production by expression in host cells that comprise the antigen-binding fragments. Examples of the antigen-binding proteins include, but are not limited to, antibodies, antigen-binding fragments, immunoconjugates, multispecific antibodies (e.g., bispecific antibodies), antibody fragments, antibody derivatives, antibody analogues, or fusion proteins, as long as they exhibit the desired antigen-binding activity.

The present invention provides the above antibody targeting CD7 and the antigen-binding fragment thereof. The antibody or the antigen-binding fragment thereof has good safety and targeting properties, and can specifically bind to the extracellular domain of human CD7. By means of infecting an immune cell with a vector comprising a coding sequence of the antibody or the antigen-binding fragment thereof, an immune cell with significant killing ability to tumour cells expressing CD7 can be obtained. The immune cell can be used for treating or ameliorating a disease related to CD7 expression, thereby laying a foundation for the treatment of CD7-positive tumours.

In certain embodiments, the antibody targeting CD7 comprises a heavy chain variable region, wherein the heavy chain variable region comprises CDR1, CDR2, and CDR3, wherein the CDR1 comprises a sequence as set forth in SEQ ID NO: 18, the CDR2 comprises a sequence as set forth in SEQ ID NO: 19, and the CDR3 comprises a sequence as set forth in SEQ ID NO: 20;

the CDR1 comprises a sequence as set forth in SEQ ID NO: 42, the CDR2 comprises a sequence as set forth in SEQ ID NO: 43, and the CDR3 comprises a sequence as set forth in SEQ ID NO: 44;
the CDR1 comprises a sequence as set forth in SEQ ID NO: 2, the CDR2 comprises a sequence as set forth in SEQ ID NO: 3, and the CDR3 comprises a sequence as set forth in SEQ ID NO: 4;
the CDR1 comprises a sequence as set forth in SEQ ID NO: 6, the CDR2 comprises a sequence as set forth in SEQ ID NO: 7, and the CDR3 comprises a sequence as set forth in SEQ ID NO: 8;
the CDR1 comprises a sequence as set forth in SEQ ID NO: 10, the CDR2 comprises a sequence as set forth in SEQ ID NO: 11, and the CDR3 comprises a sequence as set forth in SEQ ID NO: 12;
the CDR1 comprises a sequence as set forth in SEQ ID NO: 14, the CDR2 comprises a sequence as set forth in SEQ ID NO: 15, and the CDR3 comprises a sequence as set forth in SEQ ID NO: 16;
the CDR1 comprises a sequence as set forth in SEQ ID NO: 22, the CDR2 comprises a sequence as set forth in SEQ ID NO: 23, and the CDR3 comprises a sequence as set forth in SEQ ID NO: 24;
the CDR1 comprises a sequence as set forth in SEQ ID NO: 26, the CDR2 comprises a sequence as set forth in SEQ ID NO: 27, and the CDR3 comprises a sequence as set forth in SEQ ID NO: 28;
the CDR1 comprises a sequence as set forth in SEQ ID NO: 30, the CDR2 comprises a sequence as set forth in SEQ ID NO: 31, and the CDR3 comprises a sequence as set forth in SEQ ID NO: 32;
the CDR1 comprises a sequence as set forth in SEQ ID NO: 34, the CDR2 comprises a sequence as set forth in SEQ ID NO: 35, and the CDR3 comprises a sequence as set forth in SEQ ID NO: 36;
the CDR1 comprises a sequence as set forth in SEQ ID NO: 38, the CDR2 comprises a sequence as set forth in SEQ ID NO: 39, and the CDR3 comprises a sequence as set forth in SEQ ID NO: 40; or

the CDR1 comprises a sequence as set forth in SEQ ID NO: 46, the CDR2 comprises a sequence as set forth in SEQ ID NO: 47, and the CDR3 comprises a sequence as set forth in SEQ ID NO: 48;

the CDR1, the CDR2, and the CDR3 each comprise up to 3 amino acid substitutions, insertions, or deletions.

In certain embodiments, the CDR1, the CDR2, and the CDR3 each comprise up to 1 or 2 amino acid substitutions, insertions, or deletions.

In certain embodiments, the antibody or the antigen-binding fragment thereof comprises an amino acid sequence as set forth in any one of SEQ ID NOs: 13, 17, 41, and 49-51, or a mutant amino acid sequence that comprises up to 5 amino acid substitutions, insertions, or deletions in the framework region relative to the amino acid sequence. In certain embodiments, the antibody or the antigen-binding fragment thereof comprises a mutant amino acid sequence that comprises up to 5, 4, 3, 2, or 1 amino acid substitutions, insertions, or deletions in the framework region relative to an amino acid sequence as set forth in any one of SEQ ID NOs: 17, 41, 1, 5, 9, 13, 21, 25, 29, 33, 37, 45, and 49-51. In certain embodiments, the antibody or the antigen-binding fragment thereof comprises a mutant amino acid sequence that comprises up to 5, 4, 3, 2, or 1 conservative amino acid substitutions in the framework region relative to an amino acid sequence as set forth in any one of SEQ ID NOs: 13, 17, 41, and 49-51. In some embodiments, the antibody or the antigen-binding fragment thereof comprises a mutant sequence having at least 80%, 85%, 90%, 95%, 96%, 97%, 98%, or 99% identity to an amino acid sequence as set forth in any one of SEQ ID NOs: 17, 41, 1, 5, 9, 13, 21, 25, 29, 33, 37, 45, and 49-51.

In the present application, the terms "VHH" and "single domain antibody (sdAb)" generally refer to an antibody with target binding activity located in a single domain, which is also referred to as a nanobody. In the present application, the single domain antibody may be an alpaca single domain antibody. Unlike, for example, antibodies or single-chain antibodies (which typically require both heavy chain and light chain variable domains to contribute to antigen-binding activity), in certain embodiments, the antibody or the antigen-binding fragment thereof described in the present application is sdAb.

In the present application, the term "Fc region (fragment crystallizable region)", also referred to as the constant region, generally refers to the region of an antibody that corresponds to the antigen-binding region of the antibody. The Fc region generally remains constant itself and is not responsible for binding to antigens. However, it can bind to Fc receptors and complement, thereby exerting the biological functions of the antibody. The Fc region of the present application may be the Fc of a human antibody. In some examples, the Fc region may comprise a hinge, CH2, and CH3. Dimerization between two Fc-containing polypeptides may be mediated when the Fc region comprises a hinge. The Fc fragment may be derived from IgG, IgM, IgD, IgE, or IgA. In some examples, the Fc region is derived from IgG1, IgG2, IgG3, or IgG4. The "Fc fragment" also includes variant Fc fragments derived from natural Fc fragments, which have been modified while retaining their effector functions. The "variant Fc fragment" comprises an amino acid sequence having at least one amino acid alternation relative to the amino acid sequence of a natural Fc fragment. In some examples, the variant Fc fragment has at least one amino acid substitution compared to the parent Fc fragment (natural Fc fragment). For example, about 1 to about 10 amino acids, preferably about 1 to about 5 amino acids, are substituted in the parent Fc fragment. In some examples, the variant Fc fragment Fc region has at least about 80% sequence identity, at least about 90% sequence identity, at least about 95% sequence identity, at least about 96% sequence identity, at least about 97% sequence identity, at least about 98% sequence identity, or at least about 99% sequence identity to the parent Fc fragment. The effector functions of the "Fc fragment" may include binding to Fc receptors, C1q binding, complement-dependent cytotoxicity (CDC), antibody-dependent cell-mediated cytotoxicity (ADCC), mediation of phagocytosis, etc. In certain embodiments, the antibody or the antigen-binding fragment thereof of the present application comprises an Fc region, e.g., fused scFv, HcAb, full-length antibody, etc.

For an antibody or an antigen-binding fragment thereof, the term "bind", "directed against", or "specifically bind" means that a molecule (e.g., an antibody or an antigen-binding fragment thereof) has higher binding affinity for one molecule (such as an antigen) relative to other molecules present simultaneously in the environment. One molecule may bind, be directed against, or specifically bind to one or more molecules. For example, a bispecific antibody may have a higher binding affinity for two different antigens relative to other molecules. The binding affinity of an antibody for an antigen can be measured using several parameters, e.g., the EC50 value or KD value for the binding of an antibody to an antigen.

$EC_{50}$ (concentration for 50% of maximal effect) refers to the concentration that induces 50% of the maximum effect. When used in an enzyme-linked immunosorbent assay (ELISA) to indicate the binding capacity of an antibody molecule to a corresponding antigen, it may refer to the concentration of the antibody molecule at which half of the maximum detectable signal (e.g., colorimetric or fluorescent intensity) is produced. The lower the EC50 value, the greater the binding affinity of the antibody for the antigen. In certain embodiments, the EC50 value for the binding of the antibody or the antigen-binding fragment thereof to the human CD7 molecule is less than 1 nM.

The KD value may also be used to measure the binding affinity of an antibody for an antigen. The KD value is the equilibrium dissociation constant between an antibody and an antigen thereof, i.e., the ratio of koff/kon. Therefore, the lower the KD value (i.e., the lower the concentration), the higher the affinity of the antibody. In some embodiments of the present application, as detected by Biacore, the binding KD value of the antibody or the antigen-binding fragment thereof to the CD7 molecule is less than 1 nM. In some embodiments, as detected by Biacore, the binding KD value of the antibody or the antigen-binding fragment thereof to the CD7 molecule is less than 0.1 nM. In some embodiments, as detected by Biacore, the antibody or the antigen-binding fragment thereof is humanized VHH, and the binding KD value of the humanized VHH to the CD7 molecule is less than 1 nM, and more preferably, less than 0.1 nM.

In the present application, the term "blocking" generally refers to an effect on the expression of the CD7 molecule on the cell surface, for example, the downregulation of the expression of the CD7 molecule on the cell surface. In some embodiments, the blocking molecule of the present application has a blocking effect on the expression of the CD7 molecule on the surface of T cells; in some embodiments, the efficiency of the blocking effect is 70%, 80%, 90%, or 100%. In some embodiments, the blocking effect is achieved by introducing a nucleic acid encoding the blocking molecule into an immune cell. In some embodiments, the blocking effect is achieved by introducing a blocking molecule into an immune cell. In some embodiments, the blocking effect reaches 100% on or after day 5, day 6, day 7, day 8, or day 9 after the introduction of the blocking molecule or the nucleic acid encoding the blocking molecule into the cell. In some embodiments, the blocking effect can last for 10 days, 15 days, 20 days, or longer.

In certain embodiments, the amino acid sequence of the CD7 blocking molecule comprises a mutant sequence having 80%, 85%, 90%, 95%, 96%, 97%, 98%, or 99% identity to a sequence as set forth in SEQ ID NO: 52. In certain embodiments, the amino acid sequence of the CD7 blocking molecule comprises a sequence as set forth in SEQ ID NO: 52.

In the present application, the term "transmembrane domain" generally refers to a sequence in a cell surface protein that spans the cell membrane, which may contain a hydrophobic alpha helix. The transmembrane domain can be linked to the intracellular signal transduction domain and functions to transmit signals. In the present application, the transmembrane domain can be derived from any type I, type II, or type III transmembrane protein.

In the present application, the term "chimeric antigen receptor (CAR)" generally refers to a fusion protein that comprises an extracellular domain capable of binding to an antigen and at least one intracellular domain. CAR is the core component of chimeric antigen receptor T (CAR-T) cells, which may include an antigen (e.g., tumour-specific antigen and/or tumour-associated antigen) binding domain, a transmembrane domain, a co-stimulatory domain, and an intracellular signalling domain. CAR is an engineered receptor that can implant a receptor of any specificity into immune effector cells, particularly T cells. In CAR, the scFv fragment or VHH fragment of a monoclonal antibody that specifically recognizes a tumour antigen can be implanted into T cells or NK cells. The nucleic acid encoding CAR can be introduced into T cells, NK cells, or NK T cells using, for example, retroviral vectors. In this way, a large number of cancer-specific T cells, NK cells or NK T cells can be generated for adoptive cell transfer. In the present application, the CAR may be constructed by combining the antigen (e.g., BCMA) specificity of an antibody and the intracellular activation domain of a T cell receptor. T cells that have been genetically modified to express CARs can specifically recognize and eliminate malignant cells expressing the target antigen. For descriptions of CAR and CAR-T cells, reference may be made to, for example, Sadelain M, Brentjens R, Riviere I. The basic principles of chimeric antigen receptor design. Cancer Discov. 2013; 3(4): 388-398; Turtle CJ, Hudecek M, Jensen MC, Riddell SR. Engineered T cells for anti-cancer therapy. Curr Opin Immunol. 2012; 24(5): 633-639; Dotti G, Gottschalk S, Savoldo B, Brenner MK. Design and development of therapies using chimeric antigen receptor-expressing T cells. Immunol Rev 2014; 257 (1): 107-126; and WO 2013154760 and WO 2016014789.

In the present application, the term "co-stimulatory domain" generally refers to the intracellular domain of an immune co-stimulatory molecule, wherein the co-stimulatory molecule is a cell surface molecule required for the effective response of lymphocytes to antigens.

In the present application, the term "hinge region" generally refers to a connecting region between an extracellular domain (e.g., a BCMA-targeting moiety) and a transmembrane region. In the present application, the CAR may comprise one or more hinge regions between the BCMA-targeting moiety and the transmembrane domain.

In the present application, the term "signalling domain" generally refers to a domain located inside the cell that is capable of transducing signals. In the present application, the intracellular signalling domain can transduce signals into the cell. Generally, a signalling domain is any continuous amino acid sequence used for guiding a protein to find a target. In certain cases, the signalling domain may be derived from CD3ζ. CD3ζ can form a T cell receptor-CD3 complex with T cell receptor subunits and CD3-gamma, -delta, and -epsilon. CD3ζ comprises three ITAM motifs, and the ITAM sequence mediates the intracellular signal activation of the TCR. The chain is a receptor-activated protein tyrosine kinase substrate. When the TCR receptor binds to the polypeptide-MHC complex, the chain can undergo tyrosine phosphorylation and participate in the transduction of lymphocyte activation signals. Therefore, CD3ζ plays a critical role in antigen recognition and TCR signal transduction.

In the present application, the "vector" generally refers to a nucleic acid molecule capable of self-replication in a suitable host for the purpose of transferring an inserted nucleic acid molecule into a host cell and/or between host cells. The vector may include a vector primarily used for inserting DNA or RNA into a cell, a vector primarily used for replicating DNA or RNA, and a vector primarily used for the transcriptional and/or translational expression of DNA or RNA. The vector also comprises a vector having a variety of the above-mentioned functions. The vector may be a polynucleotide capable of being transcribed and translated into a polypeptide when introduced into a suitable host cell. Generally, the vector can produce the desired expression product by culturing an appropriate host cell comprising the vector. The vector can encompass additional features other than the transgenic insertion sequence and backbone: a promoter, a genetic marker, antibiotic resistance, a reporter gene, a targeting sequence, and a protein purification tag. Vectors referred to as expression vectors (expression constructs) can be used to express transgenes in target cells and generally have control sequences. The vectors of the present application may be expression vectors, which can include viral vectors (lentiviral vectors and/or retroviral vectors), phage vectors, phagemids, cosmids, artificial chromosomes such as yeast artificial chromosomes (YACs), bacterial artificial chromosomes (BACs) or P1-derived artificial chromosomes (PACs), and/or plasmids.

In the present invention, the term "engineered immune cell" refers to an immune cell that can perform immune effector functions after being artificially modified. In some embodiments, the engineered immune cell expresses at least FcγRIII and performs ADCC effector functions. Examples of immune effector cells that mediate ADCC include peripheral blood mononuclear cells (PBMCs), natural killer (NK) cells, monocytes, cytotoxic T cells, neutrophils, and eosinophils. Preferably, the immune cell is selected from; at least one of an immune cell differentiated from a pluripotent stem cell or an embryonic stem cell in culture, a T lymphocyte, an NK cell, a peripheral blood mononuclear cell (PBMC), and a hematopoietic stem cell.

More preferably, the immune cell is a T lymphocyte (also referred to as a T cell). In some embodiments, T cell may be CD4+/CD8-, CD4-/CD8+, CD4+/CD8+, CD4-/CD8-, or a combination thereof. In some embodiments, the T cell produces IL-2, IFN, and/or TNF when expressing a chimeric antigen receptor and binding to a target cell. In some embodiments, the CD8+ T cell lyses an antigen-specific target cell when expressing a chimeric antigen receptor and binding to the target cell.

The present invention provides a preparation method for the engineered immune cell, which comprises infecting an immune cell with the isolated nucleic acid or the vector of the present invention. Preferably, in the present invention, a genetically modified immune effector cell is prepared by introducing a chimeric antigen receptor into an immune effector cell (such as a T cell). In some embodiments, the preparation method comprises first introducing a nucleic acid encoding the blocking molecule into an immune cell to be modified to block the expression of a CD7 molecule, and then transfecting a nucleic acid encoding a CD7-CAR molecule on day 5, day 6, day 7, day 8, or day 9 after the introduction to express the CD7-CAR molecule on the surface of the immune cell.

It should be noted that a method for introducing a nucleic acid or vector into a mammalian cell is known in the art, and the vector can be transferred into an immune effector cell by physical, chemical, or biological methods. The physical methods for introducing the vector into the immune effector cell include calcium phosphate precipitation, lipofection, particle bombardment, microinjection, electroporation, etc. The chemical means for introducing the nucleic acid or vector into the immune effector cell include colloidal dispersion systems, such as macromolecular complexes, nanocapsules, microspheres, beads, and lipid-based systems (including oil-in-water emulsions, micelles, mixed micelles, and liposomes). The exemplary colloidal system used as an *in vitro* delivery vehicle is a liposome (e.g., an artificial membrane vesicle). Biological methods for introducing the nucleic acid or the vector into the immune effector cell include the use of DNA and RNA vectors. Viral vectors have become the most widely used method for inserting genes into mammalian cells, such as human cells. In some embodiments, the transduced or transfected immune effector cell is expanded *ex vivo* after the introduction of the nucleic acid or vector.

In some embodiments, the preparation method further comprises further evaluating or screening the transduced or transfected immune cells to select an engineered immune cell. In some embodiments, the CAR molecule further comprises a tag sequence for screening cells. In some embodiments, the tag sequence is a flag sequence; preferably, the tag sequence is a sequence as set forth in SEQ ID NO: 54.

In the present application, the term "treatment" generally refers to: (i) preventing the occurrence of a disease, disorder, and/or condition in a patient who may be predisposed to, but has not yet been diagnosed with, the disease, disorder, and/or condition; (ii) inhibiting the disease, disorder, or condition, i.e., arresting its development; and (iii) alleviating the disease, disorder, or condition, that is, causing the regression of the disease, disorder, and/or condition and/or symptoms associated therewith.

Herein, "cancer related to CD7 expression" refers to a disease directly or indirectly caused by abnormal CD7 expression, and generally refers to a disease caused by CD7 overexpression. Preferably, the cancer or tumour is a haematological malignancy. Further preferably, the haematological malignancy is a T cell-related tumour, wherein the T cell-related tumour includes leukaemia, lymphoma, and myeloma.

In the present application, the term "pharmaceutically acceptable carrier" generally refers to one or more non-toxic materials that do not interfere with the efficacy of the biological activity of the active ingredient. Such formulations may routinely comprise a salt, a buffer, a preservative, a compatible carrier, and optionally other therapeutic agents. Such pharmaceutically acceptable formulations may also comprise a compatible solid or liquid filler, diluent, or encapsulating substance suitable for administration to humans. Other contemplated carriers, excipients, and/or additives that can be used in the formulations described herein include: for example, flavouring agents, antimicrobials, sweetening agents, antioxidants, antistatic agents, lipids, protein excipients (such as serum albumin, gelatine, and casein), salt-forming counterions (such as sodium), etc.

In the present application, the term "and/or" should be understood as meaning either one of the options or both of the options.

In the present application, the term "comprise" generally means including the explicitly specified features, without excluding other elements.

In the present application, the term "about" generally refers to a variation within a range of 0.5% to 10% above or below the specified value, for example, a variation within the range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

Unless defined otherwise, or clear from the background, all technical and scientific terms used in the present invention have the same meaning as commonly understood by one of ordinary skill in the art to which the present invention belongs.

Example 1. Discovery of positive clone molecules for human CD7 single domain antibodies

1) Animal immunization and phage library construction

The antigen used was a recombinant protein of the human CD7 extracellular domain, CD7-His (sequence reference: P09564-1, amino acid sequence from position 26 to 180, fused with a His protein tag; purchased from ACROBiosystems, CD7-H52H7). Alpaca No. JSR49 was immunized subcutaneously with a 1 : 1 emulsion containing 0.5 mg of CD7-His fusion protein in Freund's complete adjuvant. Subsequently, the alpaca received booster immunizations via subcutaneous injection of a 1 : 1 emulsion containing 0.25 mg of CD7-His in Freund's incomplete adjuvant (Sigma-Aldrich) every two weeks, up to 5 times in total. The serum titre from alpaca was detected in terms of both total IgG antibodies and single domain antibodies, using Goat anti-Llama IgG (H+L) Secondary Antibody [HRP] (Annoron, NB7242) and MonoRab™ Rabbit Anti-Camelid VHH Cocktail [HRP] (GenScript, A02016) as detection secondary antibodies, respectively. The serum titre of single domain antibodies met the library construction requirements after the fourth immunization (library construction standard: when the dilution ratio is 1 : 8000, the OD450nm response value is approximately 1.0). Moreover, in order to increase library diversity, peripheral blood mononuclear cells (PBMCs) isolated from collected blood samples after the third and fourth immunizations were combined for subsequent phage library construction.

PBMC lysate samples from the third and fourth immunizations were mixed in equal proportions. Total RNA samples were extracted according to the standard operating procedure for RNA extraction. The extracted RNA was qualitatively and quantitatively analysed using gel electrophoresis and the OD260/OD280 method. RNA was reverse-transcribed into cDNA according to the instruction manual of the Primescript™ 1st Strand cDNA synthesis kit. cDNA was used as the template for the first round of PCR, which was performed to separate conventional IgG1 and single domain antibody samples. The first-round PCR products recovered by gel electrophoresis were used as the templates for PCR amplification to obtain VHH fragments. The VHH fragments and the single domain antibody phage display vector plasmid (Nanjing Probio) were digested with Sfi I restriction enzyme. The VHH fragments were then fused into the linearized vector using T4 ligase. The ligation products were transformed via electroporation, and the electroporated cells were plated on an agar plate containing ampicillin, and cultured overnight. The colonies on the plate were counted to obtain the library capacity, and single colonies were picked for sequencing to evaluate the library quality. The library capacity and quality are shown in Table 1.

Table 1. The library capacity and quality of the alpaca immune library

| Library | Capacity | Insertion rate | In-frame rate | Diversity |
|---|---|---|---|---|
| Anti-human CD7 single domain antibody phage display library | 2.4*10^9 | 100.0% | 97.70% | 95.29% |

2) Screening of lead antibody molecules specifically targeting human CD7 using alpaca immune library

The alpaca immune library described above was subjected to two rounds of cell panning with the positive cell line Jurkat and the negative cell line Jurkat/KO CD7 (with human CD7 knocked out, Nanjing Probio) to obtain the phage library eluate. The neutralized phage panning eluate was added to the prepared TG1 bacterial suspension, mixed uniformly, and then left to stand at 37°C for 45 minutes to infect the TG1 host bacteria. After sufficient infection, the bacterial suspension was serially diluted, spread onto an agar plate with relevant resistance, and incubated overnight at 37°C in an inverted position. A 96-well sterile deep-well plate aliquoted with 0.5 ml of 2YT medium (supplemented with 0.2% w/v glucose and 0.1 mg/ml ampicillin) was prepared. Single colonies cultured on the agar plate were picked into the wells using sterile pipette tips, and then cultured at 37°C with shaking at 220 rpm overnight (16-18 hours). 0.05-0.1 ml of overnight-cultured monoclonal bacterial suspension was transferred to a newly prepared sterile deep-well plate (aliquoted with 0.5 ml of 2YT medium containing ampicillin at a final concentration of 0.1 mg/ml), and cultured until the OD value reached about 0.6-0.8 (under OD600 detection conditions). Helper phages were added, mixed uniformly with shaking, and then left to stand at 37°C for 45 minutes for infection. Subsequently, 0.5 ml of 2YT medium (containing 0.1 mg/ml ampicillin and kanamycin at a final concentration of 0.05 mg/ml after addition) was supplemented, and cultured at 25°C with shaking at 220 rpm overnight (16-18 hours). The overnight-expressed bacterial suspension was centrifuged at 4000 rpm for 10 minutes to obtain the phage display expression supernatant for subsequent tests, including ELISA binding assay of post-panning monoclonal clones with the antigen protein human CD7-His protein and FACS binding assay against cellular antigens, to obtain positive single domain antibody clones.

ELISA binding assay method: Indirect ELISA was used to evaluate the binding capacity of phage-displayed antibodies in the supernatant to human CD7-related protein. The ELISA microplate was coated with CBS at 100 $\mu$l/well, and the reagent contained 1 $\mu$g/ml recombinant human CD7-His protein. Coating was performed at 4°C overnight. The plate was washed with PBS-T (0.05% Tween) and then blocked with PBS containing 3% skim milk at 300 $\mu$l/well for 1 hour at 37°C. Subsequently, the blocking solution was discarded. 50 $\mu$l of phage expression supernatant and 50 $\mu$l of 0.1% PBST were added to each plate, and then the plate was incubated at room temperature for 2 hours. The plate was washed three times with PBST, and then incubated with horseradish peroxidase-conjugated goat anti-M13 phage antibody (Sino Biological) at 100 $\mu$l/well for 45 minutes at room temperature. The plate was washed six times with PBST. Then, TMB chromogenic solution (GenScript) was added, and the plate was incubated at room temperature for 10-15 minutes in the dark. 50 $\mu$l of 1 M HCl stop solution (Sigma) was added to stop the reaction. The plate was read at 450 nm using a microplate reader.

FACS assay method: FACS binding assay was used to evaluate the binding capacity of antibodies in the supernatant to the human CD7 antigen expressed on the cell membrane of Jurkat cells. Jurkat cells expressing human CD7 (for detection) and Jurkat/KO CD7 cells (negative cells, with human CD7 knocked out) were collected, and washed three times with PBS. $2.5 \times 10^5$ test cells, 100 $\mu$l of the test supernatant, and 3.5 $\mu$g/ml biotin-labelled anti-phage antibody were added to a 96-well plate, and then the plate was incubated at 4°C for 1 hour. Subsequently, the cells were washed 3 times with PBS. 100 $\mu$l of iFluor- labelled streptavidin (Jackson, 016-600-084) was added, and then the plate was incubated at 4°C for 45 minutes. Finally, the cells were washed 3 times with PBS. The signals were read using FACS Calibur (BD).

Example 2. Variable region sequencing of positive clones and recombinant production of single domain antibodies

Positive clones were selected based on ELISA and FACS assay results. The original colonies corresponding to the positive clones were picked for bacterial liquid culture, expression plasmid extraction, PCR and monoclonal Sanger sequencing. Finally, 12 positive clones were obtained. The corresponding relationship between the clones and sequence numbers thereof is shown in Table 2 below.

Table 2. Positive clones and corresponding sequence numbers thereof

| Positive clone | Heavy chain variable region Amino acid sequence | CDR1 region Amino acid sequence | CDR2 region Amino acid sequence | CDR3 region Amino acid sequence |
|---|---|---|---|---|
| AHP19472 | SEQ ID NO: 1 | SEQ ID NO: 2 | SEQ ID NO: 3 | SEQ ID NO: 4 |
| AHP19481 | SEQ ID NO: 5 | SEQ ID NO: 6 | SEQ ID NO: 7 | SEQ ID NO: 8 |
| AHP19483 | SEQ ID NO: 9 | SEQ ID NO: 10 | SEQ ID NO: 11 | SEQ ID NO: 12 |
| AHP19484 | SEQ ID NO: 13 | SEQ ID NO: 14 | SEQ ID NO: 15 | SEQ ID NO: 16 |
| AHP19485 | SEQ ID NO: 17 | SEQ ID NO: 18 | SEQ ID NO: 19 | SEQ ID NO: 20 |
| AHP19487 | SEQ ID NO: 21 | SEQ ID NO: 22 | SEQ ID NO: 23 | SEQ ID NO: 24 |
| AHP19490 | SEQ ID NO: 25 | SEQ ID NO: 26 | SEQ ID NO: 27 | SEQ ID NO: 28 |
| AHP19494 | SEQ ID NO: 29 | SEQ ID NO: 30 | SEQ ID NO: 31 | SEQ ID NO: 32 |
| AHP19495 | SEQ ID NO: 33 | SEQ ID NO: 34 | SEQ ID NO: 35 | SEQ ID NO: 36 |

(continued)

| Positive clone | Heavy chain variable region Amino acid sequence | CDR1 region Amino acid sequence | CDR2 region Amino acid sequence | CDR3 region Amino acid sequence |
|---|---|---|---|---|
| AHP19508 | SEQ ID NO: 37 | SEQ ID NO: 38 | SEQ ID NO: 39 | SEQ ID NO: 40 |
| AHP19509 | SEQ ID NO: 41 | SEQ ID NO: 42 | SEQ ID NO: 43 | SEQ ID NO: 44 |
| AHP19511 | SEQ ID NO: 45 | SEQ ID NO: 46 | SEQ ID NO: 47 | SEQ ID NO: 48 |

A DNA fragment containing the codon-optimized heavy chain variable region was synthesized and inserted into the pcDNA3.4-Fc (HuIgG1) expression vector to form an expression plasmid.

The above plasmid was transfected into HEK293-6E cells, which were then cultured in a shake flask at 37°C for 10 days. The supernatant was collected for antibody purification. Before purification, the pipeline and Protein A column were depyrogenated with 0.2 M NaOH. The column was re-equilibrated with a buffer containing 0.05 M Tris and 1.5 M NaCl (pH = 8.0). Subsequently, the harvested cell culture supernatant was diluted at a 1 : 1 ratio with 2× the above buffer and sterilized by filtration. The filtered supernatant was incubated with the Protein A column at room temperature for 2 hours. After washing the column with 1× the above buffer, IgG was eluted using sterile 0.1 M sodium citrate (pH 3.5). The eluate was collected and neutralized with one-ninth volume of sterile 1 M Tris-HCl (pH = 9.0). Under sterile conditions, the product was buffer-exchanged into PBS (pH 7.4) to remove any residual elution buffer and concentrate the sample. After concentration, the antibody was quantified by measuring OD280nm using an extinction coefficient Ec (0.1%) of 1.43.

The purified antibody was analysed by SDS-PAGE using a 10% precast gel (GenScript) with the BioRad electrophoresis system. The gel was stained with Estain2.0 (GenScript), and the molecular size and purity were estimated by comparing the stained bands with the Protein Ladder (GenScript).

Example 3. ELISA binding assay of purified antibodies to human-, monkey-, and mouse-derived recombinant CD7 proteins

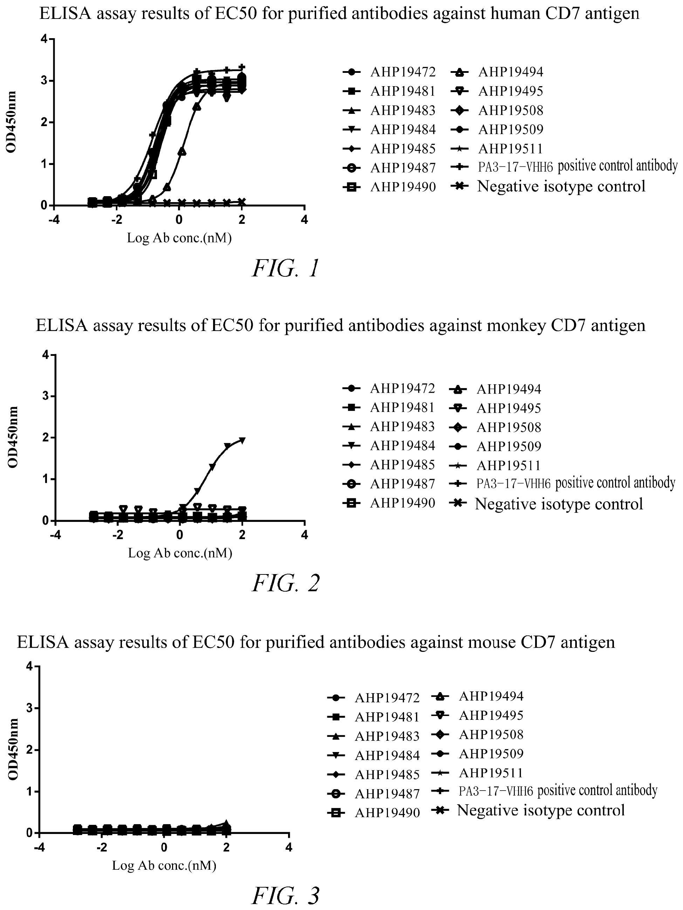

Indirect ELISA was used to evaluate the binding capacity of the purified antibodies from Example 2 to the recombinant proteins related to human CD7, monkey CD7 (Sino Biological, 90993-H08H) and mouse CD7 (ACROBiosystems, CD7-M52H3). The ELISA plates were each coated with 1 μg/ml recombinant human CD7, monkey CD7 or mouse CD7 protein in CBS at 100 μl/well overnight at 4°C. The plate was washed with PBS-T (0.05% Tween) and then blocked with PBS containing 3% skim milk at 300 μl/well for 1 hour at 37°C. Subsequently, the blocking solution was discarded. 100 μl of the purified antibody at 8 μg/ml (about 100 nM) was added to the first well, followed by 3-fold serial dilution to generate a total of 11 test concentration gradients. Then, the plate was incubated at room temperature for 1 h. The plate was washed 3 times with PBST, and then incubated with horseradish peroxidase-conjugated mouse anti-human IgG Fc fragment (Sino Biological) at 100 μl/well for 0.5 hours at 37°C. The plate was washed five times with PBST. Then, TMB chromogenic solution (GenScript) was added, and the plate was incubated at room temperature for 15 minutes in the dark. 50 μl of 1 M HCl stop solution (Sigma) was added to stop the reaction. The plate was read at 450 nm using a microplate reader. The results of the ELISA assay for the binding of 12 positive recombinant antibody clones to CD7 antigen proteins related to different species are shown in FIGs. 1-3, and the specific $EC_{50}$ values of the antibodies are listed in Table 3 below. Compared to the positive control antibody PA3-17 VHH6-Fc (HuIgG1), the binding capacity of these tested antibodies to the human CD7 antigen protein was comparable to that of the positive control antibody, with no cross-reactivity with monkey or mouse CD7.

Table 3. ELISA assay of $EC_{50}$ of purified antibodies for CD7 proteins from different species

| Purified antibody | Human CD7 protein ELISA $EC_{50}$ (nM) | Monkey CD7 protein ELISA $EC_{50}$ (nM) | Mouse CD7 protein ELISA $EC_{50}$ (nM) |
|---|---|---|---|
| AHP19472 | 0.1792 | NA | NA |
| AHP19481 | 0.2383 | NA | NA |
| AHP19483 | 0.2308 | NA | NA |
| AHP19484 | 0.2782 | 7.033 | NA |
| AHP19485 | 0.1677 | NA | NA |
| AHP19487 | 0.1734 | NA | NA |

(continued)

| Purified antibody | Human CD7 protein ELISA EC$_{50}$ (nM) | Monkey CD7 protein ELISA EC$_{50}$ (nM) | Mouse CD7 protein ELISA EC$_{50}$ (nM) |
|---|---|---|---|
| AHP19490 | 0.2676 | NA | NA |
| AHP19494 | 1.465 | NA | NA |
| AHP19495 | 0.1575 | NA | NA |
| AHP19508 | 0.2001 | NA | NA |
| AHP19509 | 0.2086 | NA | NA |
| AHP19511 | 0.1659 | NA | NA |
| PA3-17-VHH6-Fc (HuIgG1) | 0.1423 | NA | NA |
| Note: NA indicates that the purified antibody does not bind to the detected antigen protein. | | | |

Example 4. Binding of monoclonal antibodies to Jurkat cell line expressing human CD7

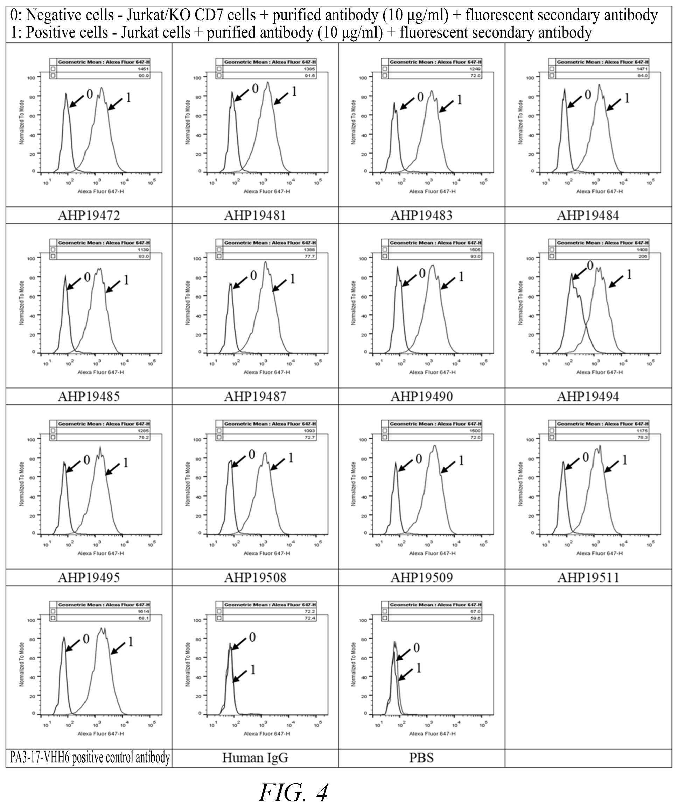

Jurkat cells expressing human CD7 (for detection) and Jurkat negative cells with human CD7 knocked out (Jurkat/KO CD7) were collected and washed 3 times with PBS. $2.5 \times 10^5$ test cells and 100 μl of the purified antibody at 10 μg/ml were added to a 96-well plate, and then the plate was incubated at 4°C for 1 hour. Subsequently, the cells were washed 3 times with PBS. 100 μl of iFluor-labelled goat anti-human IgG, Fcγ-specific fragment antibody was added, and then the plate was incubated at 4°C for 45 minutes. Finally, the cells were washed 3 times with PBS. The signals were read using FACS BD Calibur. As shown in FIG. 4, the antibodies all bound to Jurkat cells expressing human CD7 but not to the negative cells. This indicates that the 12 purified antibodies (i.e., monoclonal antibodies) all specifically bind to the human CD7 cellular antigen.

Example 5. Humanization design and recombinant production of single domain antibody molecules targeting human CD7

The structure of the parental antibody was modelled using the computer-aided homology modelling software (MOE). A human natural germline sequence with high homology to the parental antibody sequence was selected. The CDRs of the positive monoclonal antibody were grafted into the human natural germline sequence using CDR grafting technology to obtain the humanized chimeric antibody of the parental antibody, namely humanized VHH antibody. The amino acid residues of the chimeric antibody and the parental antibody were compared for differences. Key residues that might affect subsequent affinity were identified based on canonical residues, interacting loop regions, core regions, mutation hotspots, etc. Appropriate sites were selected for combinatorial design to obtain the amino acid sequences of the variable regions of the humanized antibody. The amino acid sequences of the humanized antibody were subjected to codon optimization. A DNA fragment containing the codon-optimized heavy chain variable region was synthesized and inserted into the pcDNA3.4-Fc (Human IgG1) expression vector to form an expression plasmid. The relevant expression plasmids were produced according to the methods for recombinant antibody expression and purification described in Example 2 to obtain humanized antibodies. The humanized clones and corresponding sequence numbers thereof are shown in Table 4, with CDR sequences defined according to the Kabat scheme.

Table 4. Humanized antibodies and corresponding sequence numbers thereof

| Humanized antibody | Amino acid sequence of heavy chain variable region | Amino acid sequence of CDR1 region | Amino acid sequence of CDR2 region | Amino acid sequence of CDR3 region |
|---|---|---|---|---|
| AHP 19484-VHH3 | SEQ ID NO: 49 | SEQ ID NO: 14 | SEQ ID NO: 15 | SEQ ID NO: 16 |
| AHP19485-VHH1 | SEQ ID NO: 50 | SEQ ID NO: 18 | SEQ ID NO: 19 | SEQ ID NO: 20 |
| AHP19509-VHH1 | SEQ ID NO: 51 | SEQ ID NO: 42 | SEQ ID NO: 43 | SEQ ID NO: 44 |

Example 6. SPR binding assay of humanized antibodies to human and monkey CD7 proteins

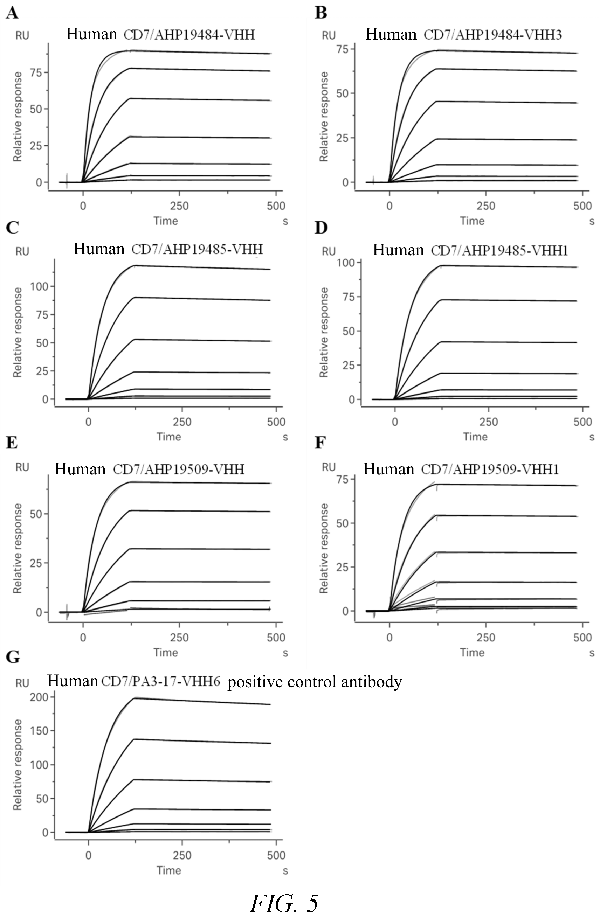

The affinities of the purified antibodies for human or monkey CD7 proteins were determined individually using a surface plasmon resonance (SPR) biosensor Biacore T200 (GE Healthcare). The antibodies were immobilized on the sensor chip using the Fc capture method. Human or monkey CD7 protein was used as an analyte. Data on dissociation (kd) and association (ka) rate constants were obtained using the Biacore T200 evaluation software. The equilibrium dissociation constant (KD) was calculated as the ratio of kd to ka. The antibodies were ranked by the equilibrium dissociation constants thereof, and the humanized antibodies with no reduction or less than a 3-fold reduction in affinity were selected for subsequent assays. The results of SPR affinity assay are shown in Tables 5-6 below, and the relevant sensorgrams are shown in FIGs. 5-6. The SPR affinity assay showed that the three groups of parental antibodies (AHP19484-VHH, AHP19485-VHH and AHP19509-VHH) and humanized antibodies (AHP19484-VHH3, AHP19485-VHH1 and AHP19509-VHH1) all exhibited comparable affinity levels for the human CD7 antigen protein, with equilibrium dissociation constants (KD) all in the $10^{-10}$ range, and none of them bound to the monkey CD7 protein.

Table 5. Affinity assay of humanized single domain antibodies for human CD7 protein

| Ligand | Analyte | Chi$^2$ (RU$^2$) | ka (1/Ms) | kd (1/s) | KD (M) | Rmax (RU) |
|---|---|---|---|---|---|---|
| AHP 19484-VHH | Human CD7 | 2.18E-01 | 5.02E+05 | 6.03E-05 | 1.20E-10 | 92.4 |
| AHP 19484-VHH3 | Human CD7 | 1.05E-01 | 4.40E+05 | 5.19E-05 | 1.18E-10 | 76.1 |
| AHP19485-VHH | Human CD7 | 6.51E-02 | 2.68E+05 | 7.73E-05 | 2.89E-10 | 122.1 |
| AHP19485-VHH1 | Human CD7 | 5.79E-02 | 2.43E+05 | 3.18E-05 | 1.31E-10 | 100.3 |
| AHP19509-VHH | Human CD7 | 9.52E-02 | 3.01E+05 | 2.30E-05 | 7.65E-11 | 68.7 |
| AHP19509-VHH1 | Human CD7 | 4.87E-01 | 3.04E+05 | 3.04E-05 | 1.00E-10 | 77.3 |
| PA3-17-VHH6 | Human CD7 | 2.88E-01 | 9.79E+04 | 1.25E-04 | 1.28E-09 | 210 |

Table 6. Affinity assay of humanized single domain antibodies for monkey CD7 protein

| Ligand | Analyte | Chi$^2$ (RU$^2$) | ka (1/Ms) | kd (1/s) | KD (M) | Rmax (RU) |
|---|---|---|---|---|---|---|
| AHP 19484-VHH3 | Monkey CD7 | NA | NA | NA | NA | NA |
| AHP19485-VHH1 | Monkey CD7 | NA | NA | NA | NA | NA |
| AHP19509-VHH1 | Monkey CD7 | NA | NA | NA | NA | NA |
| PA3-17-VHH6 | Monkey CD7 | NA | NA | NA | NA | NA |

Example 7. Exploration of antibody molecule with optimal CD7 blocking effect

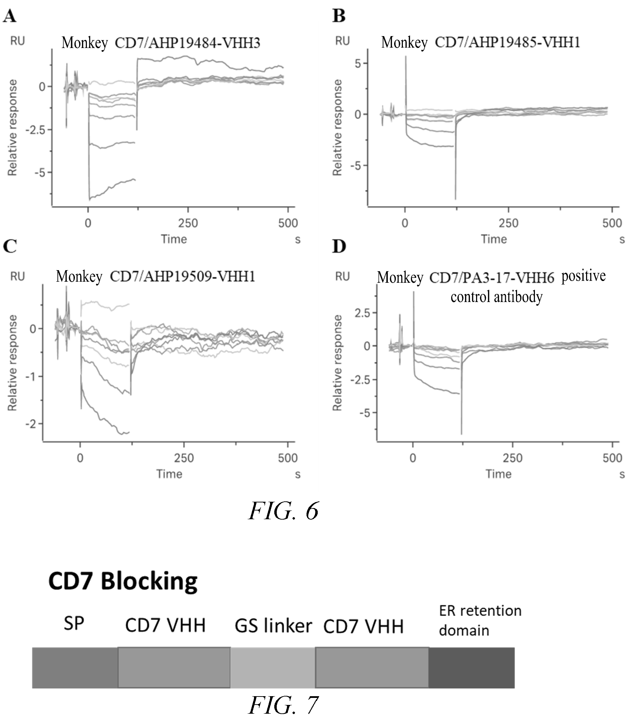

The structure of the CD7 blocking molecule is as shown in FIG. 7. Three CD7 blocking molecules with different VHH sequences were prepared. The VHH amino acid sequences are as set forth in SEQ ID NOs: 49-51. The above blocking molecules were subcloned into lentiviral vectors. The viruses carrying the CD7 blocking molecules were obtained by conventional lentivirus packaging technology for later use. The virus titre (TU/ml) was determined by the virus functional titre detection method based on flow cytometry.

Activation of T cells: The cryopreserved PBMCs (Saylibio) were thawed, and then resuspended in AIM V complete medium (Thermo Fisher, Cat# 31035025) containing 300 IU/ml rhIL-2 and counted. Subsequently, T cells were activated at a 1 : 1 ratio of cells to CD3/CD28 antibody-coated magnetic beads (GenScript, Cat# L00899-1)(the activation time point was recorded as D0, and the time points of 1 day and 2 days after activation were recorded as D1 and D2, respectively). The cells were co-incubated with the magnetic beads for 24 h to obtain activated T cells.

The activated T cells were seeded into a 24-well plate, with the cell density adjusted to $1 \times 10^6$ cells/well. Three CD7 blocking lentiviruses with different VHH sequences were added (MOI = 5 and MOI = 10), to bring the total system volume to 400 $\mu$l. Meanwhile, 16 $\mu$l of virus infection-promoting reagent HiTransG P (Genechem, Cat# REVG005) was added. 16 h after transfection, each well was supplemented with 1 ml of medium for culture. After 6 days of expansion culture, the expression of CD7 on the surface of T cells was detected by flow cytometry. The detection antibody used was: PE anti-human CD7 antibody (Biolegend, CAT# 343106).

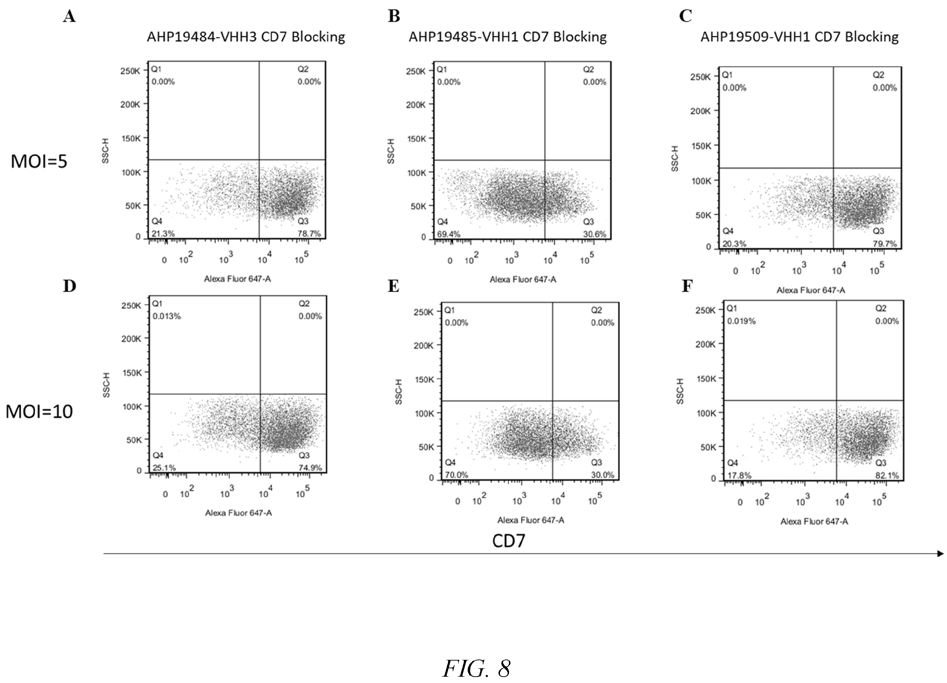

The results are as shown in FIG. 8 (in the figure, AHP19485 corresponds to the AHP19485-VHH1 molecule, AHP19484 corresponds to the AHP19484-VHH3 molecule, and AHP19509 corresponds to the AHP19509-VHH1 molecule). Under both MOI = 5 and MOI = 10 experimental conditions, the CD7 blocking effect of the molecule constructed with AHP19485-VHH1 was significantly superior to that of AHP19484-VHH3 and AHP19509-VHH1. 6 days after transfection, the blocking effect of AHP19485-VHH1 on CD7 on the surface of T cells reached 70%. Therefore, the AHP19485-VHH1 molecule was selected for subsequent construction of CD7-blocking T cells.

Example 8. Investigation of time point for blocking of CD7 molecule on the surface of T cells by CD7 blocking molecule

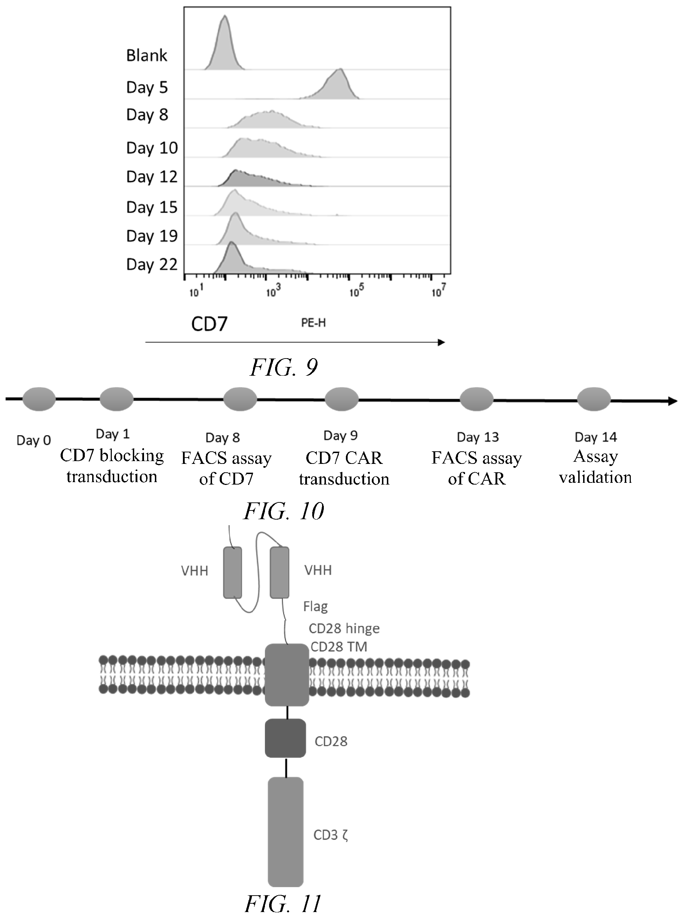

The structure of the CD7 blocking molecule is as shown in FIG. 7. The AHP19485-VHH1 molecule was used as the antibody sequence for blocking, with an amino acid sequence of SEQ ID NO: 52. The expression of CD7 on the surface of T cells was continuously monitored for 22 days by flow cytometry (detection antibody: PE anti-human CD7 Antibody, Biolegend, Cat# 343106). As shown in FIG. 9, the results showed that on day 8 after transduction with the CD7 blocking molecule, the expression level of CD7 on T cells was significantly reduced; on day 15 after transduction, the expression of 90% of CD7 molecules was blocked. This downward trend continued until day 22, with CD7 expression showing a continuous decrease, approaching 100% blocking.

The above results suggest that when preparing CD7-blocking T cells, day 8 after transduction was selected as the time point for detecting CD7 expression.

Example 9. Construction and preparation of CD7-CAR-T cells

In the present invention, a second-generation CAR vector targeting human CD7 is constructed, which sequentially comprises two repeats of the CD7 VHH antibody sequence, a Flag tag serving as a transduction indicator protein, a CD28 hinge region, a CD28 transmembrane region, a CD28 intracellular region sequence, and a CD3ζ sequence. The structure of the CAR is as shown in FIG. 11, and the amino acid sequences of the components are as set forth in SEQ ID NOs: 53-58.

To compare the differences in functional activity among 3 VHH molecule-based CAR-T cells, CD7-CAR-T cells with 3 different CD7 VHH sequences (SEQ ID NOs: 59-61) were constructed by lentiviral transfection. VHH6 CAR-T cells constructed with VHH6 (Patent No.: CN 110760007 A) were used as the positive control in the experiment (SEQ ID NO: 62). The specific construction steps were as follows:

Activation of T cells: The cryopreserved PBMCs (Saylibio) were thawed, and then resuspended in AIM V complete medium (Thermo Fisher, Cat# 31035025) containing 300 IU/ml rhIL-2 and counted. Subsequently, T cells were activated at a 1 : 1 ratio of cells to CD3/CD28 antibody-coated magnetic beads (GenScript, Cat# 31035025)(the activation time point was recorded as D0, and the time points of 1 day and 2 days after activation were recorded as D1 and D2, respectively). The cells were co-incubated with the magnetic beads for 24 h to obtain activated T cells.

Preparation of CD7-blocking T cells: The CD7 blocking molecule constructed using the AHP19485-VHH1 molecule, with an amino acid sequence of SEQ ID NO: 52, was subcloned into a lentiviral vector. The viruses carrying the CD7 blocking molecule was obtained by conventional lentivirus packaging technology for later use. The virus titre (TU/ml) was determined by the virus functional titre detection method based on flow cytometry. The activated T cells were seeded into a 24-well plate, with the cell density adjusted to $1 \times 10^6$ cells/well. The lentivirus carrying the CD7 blocking molecule (MOI = 10) was added to bring the total system volume to 400 μl. Meanwhile, 16 μl of virus infection-promoting reagent HiTransG P (Genechem, Cat# REVG005) was added. 16 h after transfection, each well was supplemented with 1 ml of medium for expansion culture. On day 8, the expression of CD7 on the surface of T cells was detected by flow cytometry.

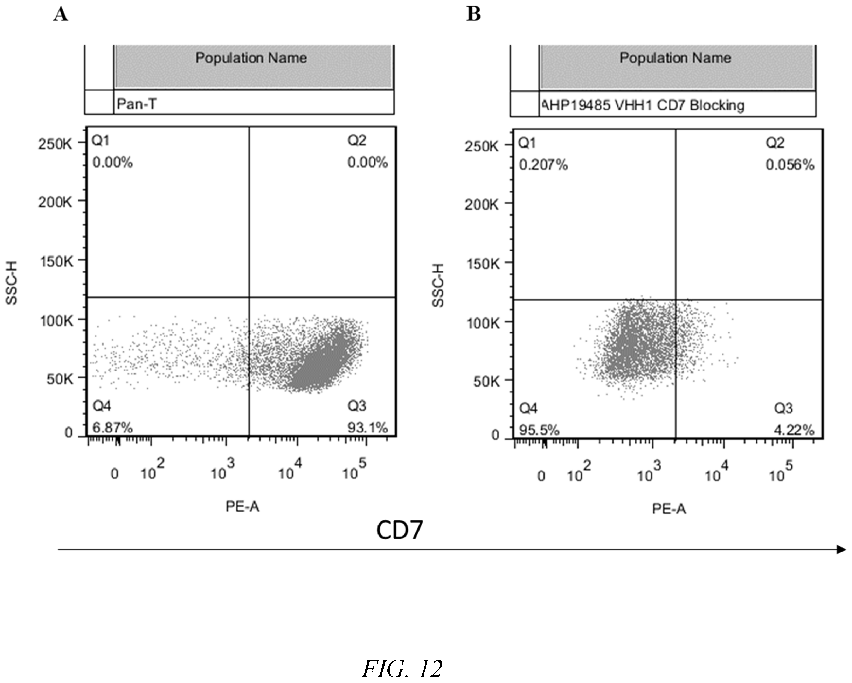

As shown in FIG. 12, the results showed that the CD7 positive rate on the surface of T cells not transduced with the virus carrying the CD7 blocking molecule (corresponding to the pan-T group in the figure) was 93.1%, whereas the CD7 positive rate in the transduced group (corresponding to the AHP19485 CD7 Blocking group in the figure) was only 4.22%, suggesting that almost no CD7 expression was detected on the surface of T cells, and the blocking effect on CD7 expression in T cells reached over 90% eight days after transduction with the virus carrying the CD7 blocking molecule. The results indicate that CD7-blocking T cells have been successfully constructed and can be used for the subsequent preparation of CD7-CAR-T cells.

Infection with lentiviral carrying CD7 CAR: On day 9, the prepared CD7-blocking T cells were seeded into a 24-well plate, with the cell density adjusted to $1 \times 10^6$ cells/well. The lentivirus carrying CD7 CAR (MOI = 10) was added to bring the total system volume to 400 μl. Meanwhile, 16 μl of virus infection-promoting reagent HiTransG P (Genechem, Cat# REVG005) was added. 16 h after transfection, each well was supplemented with 1 ml of medium for expansion culture to obtain CAR-T cells targeting CD7.

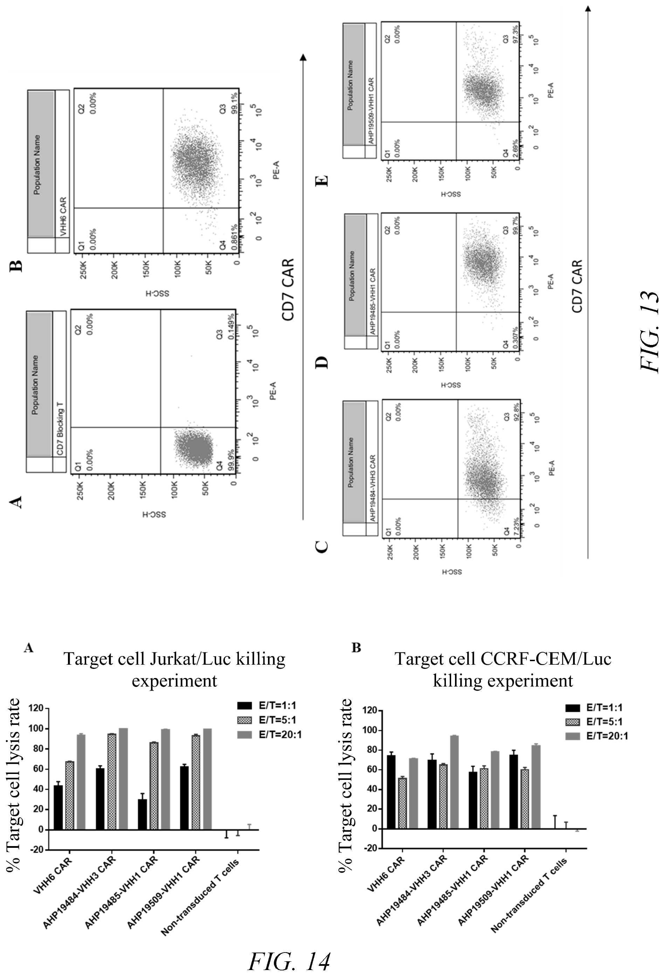

On day 13, the proportion of CD7 CAR cells was detected by flow cytometry, using PE-Labelled Human CD7 Protein, His Tag (ACROBiosystems, Cat# CD7-HP2E3) as the detection antibody. CD7 Blocking T cells, which represent T cells with blocked CD7 expression but not transduced with the virus carrying CAR, were used as the negative control; VHH6 CAR was used as the positive control. The results of CAR-positive rates are as shown in Table 7 and FIG. 13, with all CD7 CAR-positive rates exceeding 90%.

Table 7. Detection results of CD7-CAR-T positive rates

| CAR-T No. | CAR expression% |
|---|---|
| CD7 Blocking T | 0.149% |
| VHH6 CAR | 99.1% |
| AHP19484-VHH3 CAR | 92.8% |
| AHP19485-VHH1 CAR | 99.7% |
| AHP19509-VHH1 CAR | 97.3% |

Example 10. *In vitro* killing experiment of CD7-CAR-T cells against CD7-positive target cells

Target cells Jurkat/Luc (Nanjing Probio) and CCRF-CEM/Luc (Nanjing Probio) were collected, resuspended in complete medium RPMI 1640 (Gibco, Cat# 22400-089) supplemented with 10% FBS (Gibco, Cat# 10091-148), and adjusted to a density of $4 \times 10^4$ cells/mL. Then, 50 μL of the target cell suspension was transferred to a 96-well plate. CD7-CAR-T cells were collected, resuspended in complete medium RPMI 1640 (Gibco, Cat# 22400-089) supplemented with 10% FBS (Gibco, Cat# 10091-148), and adjusted to theoretical densities of $4 \times 10^4$ cells/mL (E/T = 1 : 1), $2 \times 10^5$ cells/mL (E/T = 5 : 1), and $8 \times 10^5$ cells/mL (E/T = 20 : 1), respectively, based on the CAR-positive rates of different transduced molecules (see Table 7) and various effector-to-target ratios (E/T). Then, 50 μL of the CD7-CAR-T cell suspension and 50 μL of 2% Triton X-100 (J&K Scientific Ltd., Cat# 993361) (used as the maximum killing signal value) were transferred to the 96-well plate seeded with target cells. The culture plate was then transferred to an incubator for incubation at 37°C with 5% $CO_2$ for 24 h. The culture plate after incubation for 24 h was taken out. The detection reagent Bio-Lite Luciferase Assay System (Vazyme, Cat# DD1201-02) was added, followed by incubation at room temperature for 5 minutes. Then, the luminescence signal values were detected using a microplate reader (PHERAstar FSX, BMG).

The cytotoxicity was calculated using the formula: % Cytotoxicity = 100*(1 - ($RLU_{Experimental}$ - $RLU_{Min}$)/($RLU_{UnT}$ - $RLU_{Min}$)), and the killing values of CD7-CAR-T cells with different transduced molecules were obtained. The results are as shown in FIG. 14, Table 8 and Table 9 (in the figure and tables, AHP19484 corresponds to the AHP19484-VHH3 molecule, AHP19485 corresponds to the AHP19485-VHH1 molecule, and AHP19509 corresponds to AHP19509-VHH1). When Jurkat/Luc was used as the target cell, the CAR-T cells expressing the molecular sequences AHP19484-VHH3, AHP19485-VHH1 and AHP19509-VHH1 exhibited stronger killing effects than the positive control VHH6 CAR-T cells at all three E/T ratios. When CCRF-CEM/Luc was used as the target cell, at E/T ratios of 5 : 1 and 20 : 1, the molecular sequences AHP19484-VHH3, AHP19485-VHH1 and AHP10509-VHH1 exhibited stronger killing effects than the positive control VHH6 CAR-T.

$RLU_{Experimental}$: signal value of CD7-CAR-T cells + target cells (Jurkat/Luc or CCRF-CEM/Luc);

$RLU_{UnT}$: signal value of non-transduced T cells + target cells (Jurkat/Luc or CCRF-CEM/Luc);

and

$RLU_{Min}$: signal value of 1% Triton X-100 + target cells (Jurkat/Luc or CCRF-CEM/Luc).

Table 8. Killing values of CD7-CAR-T cells against target cell Jurkat/Luc

| CD7-CAR | % Cytotoxicity | | |
|---|---|---|---|
| | E/T = 1 : 1 | E/T = 5 : 1 | E/T = 20 : 1 |
| VHH6 CAR | 43.39 | 67.15 | 93.61 |
| AHP19484 CAR | 60.49 | 94.52 | 100.00 |
| AHP19485 CAR | 29.69 | 86.04 | 99.38 |
| AHP19509 CAR | 62.44 | 92.96 | 99.63 |
| Non-transduced T cells | 0.00 | 0.00 | 0.00 |

Table 9. Killing values of CD7-CAR-T cells against target cell CCRF-CEM/Luc

| CD7-CAR | % Cytotoxicity | | |
|---|---|---|---|
| | E/T = 1 : 1 | E/T = 5 : 1 | E/T = 20 : 1 |
| VHH6 CAR | 74.48 | 51.20 | 71.11 |
| AHP19484 CAR | 69.68 | 64.90 | 94.08 |
| AHP19485 CAR | 57.39 | 61.16 | 78.24 |
| AHP19509 CAR | 74.78 | 60.22 | 84.59 |
| Non-transduced T cells | 0.00 | 0.00 | 0.00 |

The embodiments of the present invention have been described above. However, the present invention is not limited to the embodiments described above. Any modification, equivalent replacement, improvement and the like made without departing from the spirit and principle of the invention shall fall within the scope of protection of the present invention.

The information on the amino acid sequences mentioned herein is as follows.

Amino acid sequence of heavy chain variable region of AHP19472 single domain antibody: SEQ ID NO: 1

AVQLVDSGGGLVQPGGSLGLSCAASGFTFSGFDMSWYRQAPGKERM
LVATITRFGGSSNYEDSVKGRFTISRDNARDTVYLQMNSLKPEDTAMYYC
HAEAPAGSLWFGNNYWGQGTQVTVSS

Amino acid sequence of AHP19472 CDR1 region: SEQ ID NO: 2
GFTFSGFD

Amino acid sequence of AHP19472 CDR2 region: SEQ ID NO: 3
ITRFGGSS

Amino acid sequence of AHP19472 CDR3 region: SEQ ID NO: 4
HAEAPAGSLWFGNNY

Amino acid sequence of heavy chain variable region of AHP19481 single domain antibody: SEQ ID NO: 5

AVQLVDSGGGLVQPGGSLRLSCAASGFTFSGFAMSWYRQAPGKEREL
VATISRFGESSNYEDSVKGRFTISRDNAKDTGYLQMNSLKPEDTAVYYCN
AEAPAGSLWYGNNYWGQGTQVTVSS

Amino acid sequence of AHP19481 CDR1 region: SEQ ID NO: 6
GFTFSGFA

Amino acid sequence of AHP19481 CDR2 region: SEQ ID NO: 7
ISRFGESS

Amino acid sequence of AHP19481 CDR3 region: SEQ ID NO: 8
NAEAPAGSLWYGNNY

Amino acid sequence of heavy chain variable region of AHP19483 single domain antibody: SEQ ID NO: 9

AVQLVDSGGGLVQAGGSLTLSCAASGRTFSRYTMGWFRQAPGKERE
LVASISWSGGSTDYADSVKDRFTISRDNTKNTVYLQMNGLKPEDTAVYYC
NTDRMPYRPVVGGILSWEPYWGQGTQVTVTP

Amino acid sequence of AHP19483 CDR1 region: SEQ ID NO: 10
GRTFSRYT

Amino acid sequence of AHP19483 CDR2 region: SEQ ID NO: 11
ISWSGGST

Amino acid sequence of AHP19483 CDR3 region: SEQ ID NO: 12
NTDRMPYRPVVGGILSWEPY

Amino acid sequence of heavy chain variable region of AHP19484 single domain antibody: SEQ ID NO: 13

QVKLEESGGGLVQPGGSLRLSCVASGSSFHFAFMGWYRQAPGKQREL
VADISPGNSTNYADPVKGRFVISRDNAKNTVYLQMNSLKPEDTAVYFCRD
LRSWGAWGRGTQVTVSS

Amino acid sequence of AHP19484 CDR1 region: SEQ ID NO: 14
GSSFHFAF

Amino acid sequence of AHP19484 CDR2 region: SEQ ID NO: 15
ISPGNST

Amino acid sequence of AHP19484 CDR3 region: SEQ ID NO: 16
RDLRSWGA

Amino acid sequence of heavy chain variable region of AHP19485 single domain antibody: SEQ ID NO: 17

EVQLVESGGGLVQPGGSLRLSCASSERIFSIHAMGWYRQAPGKQREL
VASITIGGSTHYADSVKGRFTISRDNAKNTVYLHMNSLKPEDTAVYYCNS
MLLAGTVGGSWGQGTQVTVSS

Amino acid sequence of AHP19485 CDR1 region: SEQ ID NO: 18
ERIFSIHA

Amino acid sequence of AHP19485 CDR2 region: SEQ ID NO: 19
ITIGGST

Amino acid sequence of AHP19485 CDR3 region: SEQ ID NO: 20
NSMLLAGTVGGS

Amino acid sequence of heavy chain variable region of AHP19487 single domain antibody: SEQ ID NO: 21

DVQLVESGGGLVQPGGSLRLSCAFPGSAFSFYFMGWYRQAPGKQREL
VGDITPGGTANYADSVKGRFTISRDSAKNTVYLQMNSLKPEDTAVYYCNA
DRRVTIRPDGYWGQGTQVTVSS

Amino acid sequence of AHP19487 CDR1 region: SEQ ID NO: 22
GSAFSFYF

Amino acid sequence of AHP19487 CDR2 region: SEQ ID NO: 23
ITPGGTA

Amino acid sequence of AHP19487 CDR3 region: SEQ ID NO: 24

NADRRVTIRPDGY

Amino acid sequence of heavy chain variable region of AHP19490 single domain antibody: SEQ ID NO: 25

QVQLVESGGGLVQPGGSLRLSCAASGFTFSGFDMSWYRQAPGKEREL
VATISRFGGSSNYEDSVKGRFTISRDNVKDTVYLQMNSLKPEDTAVYYCY
ALAPAGSLWHGDNYWGQGTQVTVSS

Amino acid sequence of AHP19490 CDR1 region: SEQ ID NO: 26
GFTFSGFD

Amino acid sequence of AHP19490 CDR2 region: SEQ ID NO: 27
ISRFGGSS

Amino acid sequence of AHP19490 CDR3 region: SEQ ID NO: 28
YALAPAGSLWHGDNY

Amino acid sequence of heavy chain variable region of AHP19494 single domain antibody: SEQ ID NO: 29

QVKLEESGGGLVQAGGSLRLSCAAPGRTLSASIKAWFRQAPGKDREF
AAAIRWSGDSTYYADSVKGRFTISRDNAKNTVDLQMNSLKPEDTAVYYC
NAGGLLSNGYQPGSSWGQGTQVTVSS

Amino acid sequence of AHP19494 CDR1 region: SEQ ID NO: 30
GRTLSASI

Amino acid sequence of AHP19494 CDR2 region: SEQ ID NO: 31
IRWSGDST

Amino acid sequence of AHP19494 CDR3 region: SEQ ID NO: 32
NAGGLLSNGYQPGSS

Amino acid sequence of heavy chain variable region of AHP19495 single domain antibody: SEQ ID NO: 33

AVQLVESGGGLVQPGGSLRLSCAASGFTASRDVMSWYRQPPGKEREL
VASIFSAGSKETYAESVKGRFTISIDNVKNTVNLQMDNLKPEDTAVYYCHR
WPGYWGQGTQVTVSS

Amino acid sequence of AHP19495 CDR1 region: SEQ ID NO: 34
GFTASRDV

Amino acid sequence of AHP19495 CDR2 region: SEQ ID NO: 35
IFSAGSKE

Amino acid sequence of AHP19495 CDR3 region: SEQ ID NO: 36
HRWPGY

Amino acid sequence of heavy chain variable region of AHP19508 single domain antibody: SEQ ID NO: 37

AVQLVDSGGGLVQPGGSLRLSCAASGDFARLYEMAWYRQTPGQQRE
VVAQITVGGSTKYADSVKGRFTISRDSAKNTVYLQMNSLRPEDTAVYYCH
AWPALWGQGTQVTVSS

Amino acid sequence of AHP19508 CDR1 region: SEQ ID NO: 38
GDFARLYE

Amino acid sequence of AHP19508 CDR2 region: SEQ ID NO: 39
ITVGGST

Amino acid sequence of AHP19508 CDR3 region: SEQ ID NO: 40
HAWPAL

Amino acid sequence of heavy chain variable region of AHP19509 single domain antibody: SEQ ID NO: 41

QVKLEESGGGLVQAGGSLRLSCAASGGTFNRYTMGWFRQAPGKERE
FVAAISWSGGSTDYADSVKGRFTISRDNTKNTVYLQMNSLKPEDAAVYYC
DTDRMPYRPVAAGILSWEANWGQGTQVTVSS

Amino acid sequence of AHP19509 CDR1 region: SEQ ID NO: 42
GGTFNRYT

Amino acid sequence of AHP19509 CDR2 region: SEQ ID NO: 43
ISWSGGST

Amino acid sequence of AHP19509 CDR3 region: SEQ ID NO: 44
DTDRMPYRPVAAGILSWEAN

Amino acid sequence of heavy chain variable region of AHP19511 single domain antibody: SEQ ID NO: 45

AVQLVESGGGLVQPGGSLRLSCAASGFTFSNAVIGWYRQAPGKQREL
VASITPGGRTTYPDSVKGRFIISRDNAKNTVYLEMNSLKLEDTAVYYCNDF
RGLGGYWGQGTQVTVSS

Amino acid sequence of AHP19511 CDR1 region: SEQ ID NO: 46
GFTFSNAV

Amino acid sequence of AHP19511 CDR2 region: SEQ ID NO: 47
ITPGGRT

Amino acid sequence of AHP19511 CDR3 region: SEQ ID NO: 48
NDFRGLGGY

Amino acid sequence of heavy chain variable region of AHP19484-VHH3 single domain antibody: SEQ ID NO: 49

QVQLVESGGGLVKPGGSLRLSCAASGSSFHFAFMGWYRQAPGKQRE
LVADISPGNSTNYADPVKGRFTISRDNAKNSLYLQMNSLRAEDTAVYFCR
DLRSWGAWGQGTLVTVSS

Amino acid sequence of heavy chain variable region of AHP19485-VHH1 single domain antibody: SEQ ID NO: 50

EVQLVESGGGLVQPGGSLRLSCAASERIFSIHAMGWYRQAPGKQREL
VSSITIGGSTHYADSVKGRFTISRDNSKNTLYLQMNSLRAEDTAVYYCNSM
LLAGTVGGSWGQGTLVTVSS

Amino acid sequence of heavy chain variable region of AHP19509-VHH1 single domain antibody: SEQ ID NO: 51

EVQLVESGGGLVQPGGSLRLSCAASGGTFNRYTMGWFRQAPGKERE
FVSAISWSGGSTDYADSVKGRFTISRDNSKNTLYLQMNSLRAEDTAVYYC
DTDRMPYRPVAAGILSWEANWGQGTLVTVSS

Amino acid sequence of CD7 blocking molecule: SEQ ID NO: 52

MLLLVTSLLLCELPHPAFLLIPEVQLVESGGGLVQPGGSLRLSCAASER
IFSIHAMGWYRQAPGKQRELVSSITIGGSTHYADSVKGRFTISRDNSKNTLY
LQMNSLRAEDTAVYYCNSMLLAGTVGGSWGQGTLVTVSSGGGSGGGSG
GGSGGGSEVQLVESGGGLVQPGGSLRLSCAASERIFSIHAMGWYRQAPGK
QRELVSSITIGGSTHYADSVKGRFTISRDNSKNTLYLQMNSLRAEDTAVYY
CNSMLLAGTVGGSWGQGTLVTVSSEQKLISEEDLKDEL*

Signal peptide sequence: SEQ ID NO: 53
MLLLVTSLLLCELPHPAFLLIP

Flag tag sequence: SEQ ID NO: 54
DYKDDDDK

Hinge region sequence: SEQ ID NO: 55
IEVMYPPPYLDNEKSNGTIIHVKGKHLCPSPLFPGPSKP

Transmembrane region sequence: SEQ ID NO: 56
FWVLVVVGGVLACYSLLVTVAFIIFWV

CD28 sequence: SEQ ID NO: 57
RSKRSRLLHSDYMNMTPRRPGPTRKHYQPYAPPRDFAAYRS

CD3ζ sequence: SEQ ID NO: 58

RVKFSRSADAPAYQQGQNQLYNELNLGRREEYDVLDKRRGRDPEMG
GKPRRKNPQEGLYNELQKDKMAEAYSEIGMKGERRRGKGHDGLYQGLST
ATKDTYDALHMQALPPR

AHP19484-VHH3 CAR sequence: SEQ ID NO: 59

MLLLVTSLLLCELPHPAFLLIPQVQLVESGGGLVKPGGSLRLSCAASGS
SFHFAFMGWYRQAPGKQRELVADISPGNSTNYADPVKGRFTISRDNAKNS

LYLQMNSLRAEDTAVYFCRDLRSWGAWGQGTLVTVSSGGGSGGGSGGG
SGGGSQVQLVESGGGLVKPGGSLRLSCAASGSSFHFAFMGWYRQAPGKQ
RELVADISPGNSTNYADPVKGRFTISRDNAKNSLYLQMNSLRAEDTAVYF
CRDLRSWGAWGQGTLVTVSSAAAIEVMYPPPYLDNEKSNGTIIHVKGKHL
CPSPLFPGPSKPFWVLVVVGGVLACYSLLVTVAFIIFWVRSKRSRLLHSDY
MNMTPRRPGPTRKHYQPYAPPRDFAAYRSRVKFSRSADAPAYQQGQNQL
YNELNLGRREEYDVLDKRRGRDPEMGGKPRRKNPQEGLYNELQKDKMA
EAYSEIGMKGERRRGKGHDGLYQGLSTATKDTYDALHMQALPPR*

AHP19485-VHH1 CAR sequence: SEQ ID NO: 60

MLLLVTSLLLCELPHPAFLLIPEVQLVESGGGLVQPGGSLRLSCAASER
IFSIHAMGWYRQAPGKQRELVSSITIGGSTHYADSVKGRFTISRDNSKNTLY
LQMNSLRAEDTAVYYCNSMLLAGTVGGSWGQGTLVTVSSGGGSGGGSG
GGSGGGSEVQLVESGGGLVQPGGSLRLSCAASERIFSIHAMGWYRQAPGK
QRELVSSITIGGSTHYADSVKGRFTISRDNSKNTLYLQMNSLRAEDTAVYY
CNSMLLAGTVGGSWGQGTLVTVSSAAAIEVMYPPPYLDNEKSNGTIIHVK
GKHLCPSPLFPGPSKPFWVLVVVGGVLACYSLLVTVAFIIFWVRSKRSRLL
HSDYMNMTPRRPGPTRKHYQPYAPPRDFAAYRSRVKFSRSADAPAYQQG
QNQLYNELNLGRREEYDVLDKRRGRDPEMGGKPRRKNPQEGLYNELQKD
KMAEAYSEIGMKGERRRGKGHDGLYQGLSTATKDTYDALHMQALPPR*

AHP19509-VHH1 CAR sequence: SEQ ID NO: 61

MLLLVTSLLLCELPHPAFLLIPEVQLVESGGGLVQPGGSLRLSCAASG
GTFNRYTMGWFRQAPGKEREFVSAISWSGGSTDYADSVKGRFTISRDNSK
NTLYLQMNSLRAEDTAVYYCDTDRMPYRPVAAGILSWEANWGQGTLVT
VSSGGGSGGGSGGGSGGGSEVQLVESGGGLVQPGGSLRLSCAASGGTFNR
YTMGWFRQAPGKEREFVSAISWSGGSTDYADSVKGRFTISRDNSKNTLYL
QMNSLRAEDTAVYYCDTDRMPYRPVAAGILSWEANWGQGTLVTVSSAA
AIEVMYPPPYLDNEKSNGTIIHVKGKHLCPSPLFPGPSKPFWVLVVVGGVL
ACYSLLVTVAFIIFWVRSKRSRLLHSDYMNMTPRRPGPTRKHYQPYAPPRD
FAAYRSRVKFSRSADAPAYQQGQNQLYNELNLGRREEYDVLDKRRGRDP
EMGGKPRRKNPQEGLYNELQKDKMAEAYSEIGMKGERRRGKGHDGLYQ
GLSTATKDTYDALHMQALPPR*

VHH6 CAR sequence: SEQ ID NO: 62

MLLLVTSLLLCELPHPAFLLIPDVQLQESGGGLVQAGGSLRLSCAVSG
YPYSSYCMGWFRQAPGKEREGVAAIDSDGRTRYADSVKGRFTISQDNAK
NTLYLQMNRMKPEDTAMYYCAARFGPMGCVDLSTLSFGHWGQGTQVTV
SITGGGGSGGGGSGGGGSGGGGSDVQLQESGGGLVQAGGSLRLSCAVSG
YPYSSYCMGWFRQAPGKEREGVAAIDSDGRTRYADSVKGRFTISQDNAK
NTLYLQMNRMKPEDTAMYYCAARFGPMGCVDLSTLSFGHWGQGTQVTV
SITAAAIEVMYPPPYLDNEKSNGTIIHVKGKHLCPSPLFPGPSKPFWVLVVV
GGVLACYSLLVTVAFIIFWVRSKRSRLLHSDYMNMTPRRPGPTRKHYQPY
APPRDFAAYRSRVKFSRSADAPAYQQGQNQLYNELNLGRREEYDVLDKR
RGRDPEMGGKPRRKNPQEGLYNELQKDKMAEAYSEIGMKGERRRGKGH
DGLYQGLSTATKDTYDALHMQALPPR*

Endoplasmic reticulum localization domain sequence: SEQ ID NO: 63
EQKLISEEDLKDEL

Linker sequence: SEQ ID NO: 64
GGGSGGGSGGGSGGGS

## Claims

1. An antibody or an antigen-binding fragment thereof that specifically binds to human CD7, **characterised in that** the antibody or the antigen-binding fragment thereof comprises a heavy chain variable region, the heavy chain variable region comprises CDR1, CDR2, and CDR3, wherein

   the CDR1 comprises a sequence as set forth in SEQ ID NO: 18, the CDR2 comprises a sequence as set forth in SEQ ID NO: 19, and the CDR3 comprises a sequence as set forth in SEQ ID NO: 20;
   the CDR1 comprises a sequence as set forth in SEQ ID NO: 42, the CDR2 comprises a sequence as set forth in SEQ ID NO: 43, and the CDR3 comprises a sequence as set forth in SEQ ID NO: 44;
   the CDR1 comprises a sequence as set forth in SEQ ID NO: 2, the CDR2 comprises a sequence as set forth in SEQ ID NO: 3, and the CDR3 comprises a sequence as set forth in SEQ ID NO: 4;
   the CDR1 comprises a sequence as set forth in SEQ ID NO: 6, the CDR2 comprises a sequence as set forth in SEQ ID NO: 7, and the CDR3 comprises a sequence as set forth in SEQ ID NO: 8;
   the CDR1 comprises a sequence as set forth in SEQ ID NO: 10, the CDR2 comprises a sequence as set forth in SEQ ID NO: 11, and the CDR3 comprises a sequence as set forth in SEQ ID NO: 12;
   the CDR1 comprises a sequence as set forth in SEQ ID NO: 14, the CDR2 comprises a sequence as set forth in SEQ ID NO: 15, and the CDR3 comprises a sequence as set forth in SEQ ID NO: 16;
   the CDR1 comprises a sequence as set forth in SEQ ID NO: 22, the CDR2 comprises a sequence as set forth in SEQ ID NO: 23, and the CDR3 comprises a sequence as set forth in SEQ ID NO: 24;
   the CDR1 comprises a sequence as set forth in SEQ ID NO: 26, the CDR2 comprises a sequence as set forth in SEQ ID NO: 27, and the CDR3 comprises a sequence as set forth in SEQ ID NO: 28;
   the CDR1 comprises a sequence as set forth in SEQ ID NO: 30, the CDR2 comprises a sequence as set forth in SEQ ID NO: 31, and the CDR3 comprises a sequence as set forth in SEQ ID NO: 32;
   the CDR1 comprises a sequence as set forth in SEQ ID NO: 34, the CDR2 comprises a sequence as set forth in SEQ ID NO: 35, and the CDR3 comprises a sequence as set forth in SEQ ID NO: 36;
   the CDR1 comprises a sequence as set forth in SEQ ID NO: 38, the CDR2 comprises a sequence as set forth in SEQ ID NO: 39, and the CDR3 comprises a sequence as set forth in SEQ ID NO: 40; or
   the CDR1 comprises a sequence as set forth in SEQ ID NO: 46, the CDR2 comprises a sequence as set forth in SEQ ID NO: 47, and the CDR3 comprises a sequence as set forth in SEQ ID NO: 48;
the CDR1, the CDR2, and the CDR3 each comprise at most 3 amino acid mutations, insertions, or deletions.

2. The antibody or the antigen-binding fragment thereof according to claim 1, **characterised in that** the CDR region sequences are selected from any one of the following groups:

the CDR1 comprises a sequence as set forth in SEQ ID NO: 18, the CDR2 comprises a sequence as set forth in SEQ ID NO: 19, and the CDR3 comprises a sequence as set forth in SEQ ID NO: 20;
the CDR1 comprises a sequence as set forth in SEQ ID NO: 42, the CDR2 comprises a sequence as set forth in SEQ ID NO: 43, and the CDR3 comprises a sequence as set forth in SEQ ID NO: 44;
the CDR1 comprises a sequence as set forth in SEQ ID NO: 14, the CDR2 comprises a sequence as set forth in SEQ ID NO: 15, and the CDR3 comprises a sequence as set forth in SEQ ID NO: 16.

3. The antibody or the antigen-binding fragment according to claim 1, **characterised in that** the heavy chain variable region comprises a framework region of an alpaca antibody or a human antibody.

4. The antibody or the antigen-binding fragment according to claim 1, **characterised in that** the antibody or the antigen-binding fragment thereof is selected from the group consisting of a whole antibody, Fab, Fab', an Fv fragment, scFv, di-scFv, and a single domain antibody; preferably, the antibody or the antigen-binding fragment thereof is a single domain antibody.

5. The antibody or the antigen-binding fragment according to claim 1, **characterised in that** the antibody or the antigen-binding fragment thereof is a chimeric antibody, an Fc fusion antibody, or a humanized antibody.

6. The antibody or the antigen-binding fragment according to claim 5, **characterised in that** the antibody or the antigen-binding fragment thereof comprises: an amino acid sequence as set forth in any one of SEQ ID NOs: 17, 41, 1, 5, 9, 13, 21, 25, 29, 33, 37, 45, and 49-51, or a mutant amino acid sequence having at least 80% identity thereto.

7. A CD7 blocking molecule, **characterised in that** the CD7 blocking molecule comprises the antibody or the antigen-binding fragment thereof according to any one of claims 1-6 and an endoplasmic reticulum localization domain, and is capable of reducing the expression of a CD7 molecule on the cell surface.

8. The CD7 blocking molecule according to claim 7, **characterised in that** the CD7 blocking molecule comprises two identical single domain antibodies, which are connected via a linker.

9. The blocking molecule according to claim 7, **characterised in that** the sequence of the endoplasmic reticulum localization domain comprises a sequence as set forth in SEQ ID NO: 63.

10. The CD7 blocking molecule according to claim 7, **characterised in that** the CD7 blocking molecule further comprises a signal peptide sequence; preferably, the signal peptide sequence comprises a sequence as set forth in SEQ ID NO: 53.

11. The CD7 blocking molecule according to claim 7, **characterised in that** the amino acid sequence of the CD7 blocking molecule comprises a sequence as set forth in SEQ ID NO: 52 or a mutant sequence having at least 80% identity thereto.

12. A chimeric antigen receptor, **characterised in that** the chimeric antigen receptor comprises:

1) an antigen-binding domain that recognizes a CD7 antigen, wherein the antigen-binding domain comprises the antibody or the antigen-binding fragment thereof according to any one of claims 1-6;
2) a transmembrane domain; and
3) an intracellular signalling domain.

13. The chimeric antigen receptor according to claim 12, **characterised in that** the chimeric antigen receptor further comprises an intracellular co-stimulatory signalling domain.

14. The chimeric antigen receptor according to claim 12, **characterised in that** the chimeric antigen receptor sequence comprises an amino acid sequence as set forth in any one of SEQ ID NOs: 59-61.

15. An isolated nucleic acid molecule, **characterised in that** the isolated nucleic acid encodes the antibody or the antigen-binding fragment thereof according to any one of claims 1-6, or the blocking molecule according to any one of claims 7-11, or the chimeric antigen receptor according to any one of claims 12-14.

16. A vector, **characterised in that** the vector comprises the nucleic acid molecule according to claim 15.

17. A host cell, **characterised in that** the host cell comprises the vector according to claim 16.

18. An engineered immune cell, **characterised in that** the engineered immune cell comprises the antibody or the antigen-binding fragment thereof according to any one of claims 1-6, or the blocking molecule according to any one of claims 7-11, or the chimeric antigen receptor according to any one of claims 12-14.

19. The engineered immune cell according to claim 18, **characterised in that** the immune cell expresses the chimeric antigen receptor according to any one of claims 12-14.

20. A pharmaceutical composition, **characterised in that** the composition comprises the antibody or the antigen-binding fragment thereof according to any one of claims 1-6, or the blocking molecule according to any one of claims 7-11, or the chimeric antigen receptor according to any one of claims 12-14, or the engineered immune cell according to claim 18 or 19, and/or a pharmaceutically acceptable carrier.

21. The antibody or the antigen-binding fragment thereof according to any one of claims 1-6, the blocking molecule according to any one of claims 7-11, the engineered immune cell according to claim 18 or 19, or the composition according to claim 20 for use in the treatment of a tumour or cancer.

22. The use according to claim 21, **characterised in that** the cancer or tumour is a haematological malignancy; preferably, the haematological malignancy is a T cell-related tumour.

23. A method for preparing an engineered immune cell, **characterised in that** the method comprises the following steps:

(1) providing an immune cell to be used for expressing a chimeric antigen receptor, wherein the immune cell comprises the blocking molecule according to any one of claims 7-11; and
(2) introducing a nucleic acid molecule into the cell obtained in step (1), wherein the nucleic acid molecule encodes the chimeric antigen receptor according to any one of claims 12-14.

ELISA assay results of EC50 for purified antibodies against human CD7 antigen

*FIG. 1*

ELISA assay results of EC50 for purified antibodies against monkey CD7 antigen

*FIG. 2*

ELISA assay results of EC50 for purified antibodies against mouse CD7 antigen

*FIG. 3*

FIG. 4

A  Human CD7/AHP19484-VHH

B  Human CD7/AHP19484-VHH3

C  Human CD7/AHP19485-VHH

D  Human CD7/AHP19485-VHH1

E  Human CD7/AHP19509-VHH

F  Human CD7/AHP19509-VHH1

G  Human CD7/PA3-17-VHH6 positive control antibody

*FIG. 5*

**A**

Monkey CD7/AHP19484-VHH3

**B**

Monkey CD7/AHP19485-VHH1

**C**

Monkey CD7/AHP19509-VHH1

**D**

Monkey CD7/PA3-17-VHH6 positive control antibody

*FIG. 6*

## CD7 Blocking

SP     CD7 VHH     GS linker    CD7 VHH    ER retention domain

*FIG. 7*

*FIG. 8*

*FIG. 9*

| Day 0 | Day 1 CD7 blocking transduction | Day 8 FACS assay of CD7 | Day 9 CD7 CAR transduction | Day 13 FACS assay of CAR | Day 14 Assay validation |

*FIG. 10*

*FIG. 11*

*FIG. 12*

EP 4 748 858 A1

*FIG. 13*

*FIG. 14*

## INTERNATIONAL SEARCH REPORT

| International application No. |
| --- |
| **PCT/CN2024/106402** |

**A. CLASSIFICATION OF SUBJECT MATTER**

C07K 16/28(2006.01)i; C07K 19/00(2006.01)i; C12N 15/13(2006.01)i; C12N 15/62(2006.01)i; C12N 5/10(2006.01)i; A61K 39/00(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07K; C12N; A61K; A61P

Documentation searched other than minimum documentation to the extent that such documents are included in the fields searched

Electronic data base consulted during the international search (name of data base and, where practicable, search terms used)

CNKI, CNABS, CNTXT, DWPI, EPTXT, WOTXT, USTXT, ELSEVIER, ISI Web of Knowledge, PubMed, Bing, 万方, WANFANG, 百度, Baidu, NCBI, STN: 单域抗体, VHH, volatile halogenated hydrocarbon chain, 纳米抗体, nano antibody, 人 CD7, human CD7, SEQ ID NOs: 17-20

**C. DOCUMENTS CONSIDERED TO BE RELEVANT**

| Category* | Citation of document, with indication, where appropriate, of the relevant passages | Relevant to claim No. |
| --- | --- | --- |
| PX | CN 117362437 A (NANJING PROBIO BIOTECHNOLOGY CO., LTD.) 09 January 2024 (2024-01-09) <br> claims 1-23 | 1-10 (in part), 11, 12-23 (in part) |
| A | CN 115806618 A (HEBEI SENLANG BIOTECHNOLOGY CO., LTD.) 17 March 2023 (2023-03-17) <br> claims 1-10, and description, embodiment 6, and SEQ ID NOs: 3, 5 and 7 | 1-10 (in part), 11, 12-23 (in part) |
| A | CN 114591444 A (BEIJING MEIKANG GENO-IMMUNE BIOTECHNOLOGY CO., LTD.) 07 June 2022 (2022-06-07) <br> entire document | 1-10 (in part), 11, 12-23 (in part) |
| A | CN 116003631 A (JIANGSU YUANSHENG BIOLOGICAL TECHNOLOGY CO., LTD.) 25 April 2023 (2023-04-25) <br> entire document | 1-10 (in part), 11, 12-23 (in part) |
| A | US 2017226204 A1 (PERSONGEN BIOMEDICINE (SUZHOU) CO., LTD.) 10 August 2017 (2017-08-10) <br> entire document | 1-10 (in part), 11, 12-23 (in part) |

☑ Further documents are listed in the continuation of Box C. ☑ See patent family annex.

| | |
| --- | --- |
| \* Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **23 September 2024** | **08 October 2024** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/106402**

| C. | DOCUMENTS CONSIDERED TO BE RELEVANT | |
|---|---|---|
| Category* | Citation of document, with indication, where appropriate, of the relevant passages | Relevant to claim No. |
| A | US 2022324968 A1 (KYMAB LIMITED) 13 October 2022 (2022-10-13)<br>entire document | 1-10 (in part), 11,<br>12-23 (in part) |

Form PCT/ISA/210 (second sheet) (July 2022)

## INTERNATIONAL SEARCH REPORT

| International application No. |
|---|
| **PCT/CN2024/106402** |

**Box No. I          Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a.  ☑  forming part of the international application as filed.

   b.  ☐  furnished subsequent to the international filing date for the purposes of international search (Rule 13*ter*.1(a)),

   ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2024/106402**

| Box No. III | Observations where unity of invention is lacking (Continuation of item 3 of first sheet) |

This International Searching Authority found multiple inventions in this international application, as follows:

Invention 1: the technical solutions of claims 1-10 (in part), 11 and 12-23 (in part) relating to an antibody specifically binding to human CD7, of which a heavy chain variable region contains CDR1-3 with amino acid sequences as shown in SEQ ID NOs: 18-20, or an antigen-binding fragment thereof, and a related CD7 blocking molecule, chimeric antigen receptor, isolated nucleic acid molecule, vector, host cell, engineered immune cell, pharmaceutical composition, pharmaceutical use and method for preparing engineered immune cells.

Inventions 2-3: the technical solutions of claims 1-10 (in part) and 12-23 (in part) relating to antibodies specifically binding to human CD7, of which heavy chain variable regions contain CDR1-3 with amino acid sequences as shown in SEQ ID NOs: 42-44 and 14-16, or antigen-binding fragments thereof, and related CD7 blocking molecules, chimeric antigen receptors, isolated nucleic acid molecules, vectors, host cells, engineered immune cells, pharmaceutical compositions, pharmaceutical uses and methods for preparing engineered immune cells.

Inventions 4-12: the technical solutions of claims 1 (in part), 3-10 (in part), 12-13 (in part) and 15-23 (in part) relating to antibodies specifically binding to human CD7, of which heavy chain variable regions contain CDR1-3 with amino acid sequences as shown in SEQ ID NOs: 2-4, 6-8, 10-12, 22-24, 26-28, 30-32, 34-36, 38-40 and 46-48, or antigen-binding fragments thereof, and related CD7 blocking molecules, chimeric antigen receptors, isolated nucleic acid molecules, vectors, host cells, engineered immune cells, pharmaceutical compositions, pharmaceutical uses and methods for preparing engineered immune cells.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-10 (in part), 11, 12-23 (in part)**

**Remark on Protest** ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/CN2024/106402**

| Patent document cited in search report | | | Publication date (day/month/year) | Patent family member(s) | | | Publication date (day/month/year) |
|---|---|---|---|---|---|---|---|
| CN | 117362437 | A | 09 January 2024 | None | | | |
| CN | 115806618 | A | 17 March 2023 | None | | | |
| CN | 114591444 | A | 07 June 2022 | None | | | |
| CN | 116003631 | A | 25 April 2023 | None | | | |
| US | 2017226204 | A1 | 10 August 2017 | US | 10106609 | B2 | 23 October 2018 |
| | | | | WO | 2015184941 | A1 | 10 December 2015 |
| US | 2022324968 | A1 | 13 October 2022 | EP | 3956034 | A1 | 23 February 2022 |
| | | | | GB | 201905552 | D0 | 05 June 2019 |
| | | | | JP | 2022529350 | A | 21 June 2022 |
| | | | | WO | 2020212710 | A1 | 22 October 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310906380
- WO 2013154760 A
- WO 2016014789 A
- CN 110760007 A

**Non-patent literature cited in the description**

- **GOMES-SILVA D ; SRINIVASAN M ; SHARMA S ; LEE CM ; WAGNER DL ; DAVIS TH et al.** CD7-edited T cells expressing a CD7-specific CAR for the therapy of T-cell malignancies. *Blood.*, 2017, vol. 130 (3), 285-296
- **SEMPOWSKI GD ; LEE DM ; KAUFMAN RE ; HAYNES BF**. Structure and function of the CD7 molecule. *Crit Rev Immunol.*, 1999, vol. 19 (4), 331-348
- **SAFARZADEH KOZANI P ; SAFARZADEH KOZANI P ; RAHBARIZADEH F**. CAR-T cell therapy in T-cell malignancies: Is success a low-hanging fruit?. *Stem Cell Res Ther.*, 07 October 2021, vol. 12 (1), 527
- **SAMBROOK et al.** Molecular Cloning: A Laboratory Manual. Cold Spring Harbor Laboratory Press, 1989
- **SADELAIN M ; BRENTJENS R ; RIVIERE I**. The basic principles of chimeric antigen receptor design. *Cancer Discov*, 2013, vol. 3 (4), 388-398
- **TURTLE CJ ; HUDECEK M ; JENSEN MC ; RIDDELL SR**. Engineered T cells for anti-cancer therapy. *Curr Opin Immunol.*, 2012, vol. 24 (5), 633-639
- **DOTTI G ; GOTTSCHALK S ; SAVOLDO B ; BRENNER MK**. Design and development of therapies using chimeric antigen receptor-expressing T cells. *Immunol Rev*, 2014, vol. 257 (1), 107-126